# EUROPEAN PATENT APPLICATION

(11) **EP 3 113 044 A1**
(43) Date of publication of application: **04.01.2017**
(21) Application number: 15752380.4
(22) Date of filing: 23.02.2015
(51) Int. Cl.: G06F 17/30

(54) **CELL OBSERVATION INFORMATION PROCESSING SYSTEM, CELL OBSERVATION INFORMATION PROCESSING METHOD, CELL OBSERVATION INFORMATION PROCESSING PROGRAM, RECORDING UNIT PROVIDED TO CELL OBSERVATION INFORMATION PROCESSING SYSTEM, AND DEVICE PROVIDED TO CELL OBSERVATION INFORMATION PROCESSING SYSTEM**

(30) Priority: 24.02.2014 JP 2014033319
(71) Applicant: Olympus Corporation, Tokyo 192-8507 (JP)
(72) Inventor: IGA Yasunobu, Tokyo 192-8507 (JP); TANIKAWA Yohei, Tokyo 192-8507 (JP); TAKIMOTO Shinichi, Tokyo 192-8507 (JP)
(74) Representative: Schicker, Silvia
(86) International application number: PCT/JP2015/054953
(87) International publication number: WO 2015/125954

(57) **Abstract**

[Object]

To provide a cell observation information processing system that can save, as much as possible, operation effort and time of users taken to acquire tags to be assigned to observation information on cells.

[Measures for solution]

The system includes a tag acquiring section 11 that acquires certain tags, as search tags for searching for observation information acquired by an observation information acquiring section 20 in response to an acquisition order at one time point; a storing section 12 that stores, into an archive section 13, respective sets of the observation information acquired at respective time points upon assigning to them the search tags; a retrieving section 15 that retrieves a list of tags as possible search tags from the archive section; and an output section 19 that outputs the list of tags to a presenting section 18. The retrieving section designates a narrowing-down criterion for narrowing down tags to be retrieved and retrieves the list of tags as a result of narrowing down with the designated narrowing-down criterion. The tag acquiring section acquires a tag selected by the user from the list of tags presented by the presenting section.

## Description

### TECHNICAL FIELD

The present invention relates to a cell observation information processing system, a cell observation information processing method, a cell observation information processing program, an archive section provided for the cell observation information processing system, and apparatuses provided for the cell observation information processing system, each of which is to be used for condition management of cells to be used for research of living object upon use of observation information (images and activity data) on the cells acquired by an observation information acquiring apparatus such as a microscope.

### BACKGROUND ART

In the fields where experiment and research using cells are to be made, users continue culturing cells on a daily basis and control, through observation of cell images via microscopes, conditions of the cells to be used for the experiment and research.

Conventionally, cell conditions have been recognized by eye observation, through microscopes, of cells in culture containers by users. Finding variations of the cell conditions has been entrusted to memory and sense of users. However, entrusting it to the users' memory and sense would hinder accurate recognition of a long-term variation of the cell conditions, to lead to failure in enhancing accuracy of experiment and research. For accurate recognition of long-term variations of cell conditions, it is necessary to make observation information at individual time points searchable by compiling it into a database.

Thus, there have been proposed apparatuses having image databases in which and through which observation data can be stored and searched, as in the following Patent Document 1, for example.

The apparatus described in Patent Document 1 is configured so that it makes it possible to store, in a certain relational database, observation images containing images of living specimens and information on acquisition conditions at the times of capturing the observation images, such as photographing conditions and photographing date/time, to designate the information on acquisition conditions as a search criterion, and to choose and display images that are matched with the search criterion.

### PRIOR ART DOCUMENTS

### PATENT DOCUMENTS

Patent Document 1: US Patent Appln. Pub. No. 2006/0206482

### SUMMARY OF THE INVENTION

### PROBLEMS TO BE SOLVED BY THE INVENTION

In a case where observation information on cells at respective time points is compiled into a database for condition management of the cells used for experiment and research, tag items for classifying the observation information on cells are counted to be numerous. In addition, there are a plurality of image shots captured at one time point. Therefore, it takes great effort and time for a user to manually input, via a keyboard, tags to be assigned to the observation information.

For example, if the user inputs a tag using keys of letters, numerals and symbols of a keyboard, the troublesome operation easily induces misinput of the tag. Moreover, in a case of the tag having a long string of characters, inputting the tag, which involves a lot of key strokes, takes a substantial time depending on the length of the string, as well as results in increased misinputs, to require an extra time to correct each error.

Furthermore, of tag items to be assigned to observation information on cells, there are many items that are difficult to automatically assign via the apparatus but require input operation via a user, such as cell name.

In particular, in a case where tags to be assigned to observation information are inputted while living cells are taken out of the incubator and observed via an image acquiring apparatus, if it takes a long time to input the tags, the living cells are seriously damaged. As a result, the cell conditions cannot be accurately recognized and thus it comes to be difficult to ensure accurateness of experiment results.

That is, when taken out from the incubator for the purpose of acquiring observation information, the living cells, which have been cultured inside the incubator, are damaged by a change in environment including temperature and humidity, as the stress. Therefore, for accurately recognizing the cell conditions, it is important to shorten the time for which the living cells are off the incubator as much as possible.

However, Patent Document 1 merely discloses that information on acquisition conditions, which correspond to a tag, is manually inputted by a user; there is no disclosure of detailed method for manually inputting the tag.

Therefore, in fields where cell information is compiled into a database for condition management of cells used for biological research, there is still a room for improvement in reduction of time taken to acquire tags by streamlining users' operation method.

A few aspects of the present invention are proposed to solve the above-described conventional problems; an object of the present invention is to provide a cell observation information processing system, a cell observation information processing method, a cell observation information processing program, an archive section provided for the cell observation information processing system, and an apparatus provided for the cell observation information processing system, each of which can save, as much as possible, operation effort and time of the user taken to acquire tags to be assigned to observation information on cells.

### MEASURES TO SOLVE THE PROBLEMS

To attain the above-mentioned objects, a cell observation information processing system according to one aspect of the present invention includes: a tag acquiring section that acquires, as search tags for searching for observation information that has been acquired via an observation information acquiring section in response to an acquisition order at one time point to contain items carrying cell observation results such as a cell image, activity data indicating cell activity by a number of cells, a cell confluency, a morphology, a viability or so., and an observation title, at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; a storing section that assigns, as search tags, the tags acquired by the tag acquiring section to the observation information, which has been acquired in response to an acquisition order at each time point, and that stores search-tagged observation information, to which the search tags have been assigned, in an archive section; a retrieving section that retrieves from the archive section a list of tags as possible search tags; and an output section that outputs the list of tags retrieved by the retrieving section to a presenting section; wherein the retrieving section designates a narrowing-down criterion for narrowing down tags to be retrieved and retrieves the list of tags as a result of narrowing down with the designated narrowing-down criterion, and the tag acquiring section acquires a tag selected by the user from the list of tags presented by the presenting section.

A cell observation information processing method according to another aspect of the present invention includes: a tag acquiring step for acquiring, as search tags for searching for observation information that has been acquired in response to an acquisition order at one time point to contain items carrying cell observation results such as a cell image, activity data indicating cell activity by a number of cells, a cell confluency, a morphology, a viability or so., and an observation title, at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; a storing step for assigning, as search tags, the tags acquired at the tag acquiring step to the observation information, which has been acquired in response to an acquisition order at each time point, and for storing search-tagged observation information, to which the search tags have been assigned, in an archive section; a retrieving step for retrieving from the archive section a list of tags as possible search tags; and an output step for outputting the list of tags retrieved by the retrieving section to a presenting section; wherein the retrieving step includes designating a narrowing-down criterion for narrowing down tags to be retrieved and retrieving the list of tags as a result of narrowing down with the designated narrowing-down criterion, and the tag acquiring step includes acquiring a tag selected by the user from the list of tags presented by the presenting section.

A cell observation information processing program according to still another aspect of the present invention makes a computer function as: a tag acquiring means that acquires, as search tags for searching for observation information that has been acquired via an observation information acquiring section in response to an acquisition order at one time point to contain items carrying cell observation results such as a cell image, activity data indicating cell activity by a number of cells, a cell confluency, a morphology, a viability or so., and an observation title, at least one of tags of user identifying information for identifying a user who conducts cell observation, cell identifying information for identifying cells under observation, date/time information for identifying a date/time when the observation information was acquired, information on user' s work for cell maintenance, apparatus identifying information for identifying an apparatus used in acquiring the observation information, activity data variation information and an image tag for indicating the cell image; a storing means that assigns, as search tags, the tags acquired by the tag acquiring section to the observation information, which has been acquired in response to an acquisition order at each time point, and that stores search-tagged observation information, to which the search tags have been assigned, in an archive section; a retrieving means that retrieves from the archive section a list of tags as possible search tags; and an output means that outputs the list of tags retrieved by the retrieving section to a presenting section; wherein the retrieving means designates a narrowing-down criterion for narrowing down tags to be retrieved and retrieves the list of tags as a result of narrowing down with the designated narrowing-down criterion, and the tag acquiring means acquires a tag selected by the user from the list of tags presented by the presenting section.

### BRIEF DESCRIPTION OF THE DRAWINGS

FIG. 1 is a block diagram that schematically shows the basic configuration of the cell observation information processing system according to the present invention.
FIG. 2 is an explanatory diagram that shows the configuration of the cell observation information processing system according to the first embodiment mode of the present invention.
FIG. 3 is an explanatory diagram that shows one example of data structure of search-tagged observation information stored by the storing section in the archive section in the cell observation information processing system of FIG. 2.
FIG. 4. is an explanatory diagram that shows one layout example of the presenting section displaying a list of search tags retrieved by the retrieving section and outputted by the output section in the cell observation information processing system of FIG. 2.
FIG. 5. is a block diagram that shows one example of the procedure from narrowing down and retrieval of a list of search tags up to display of the list of search tags as narrowed down and retrieved.

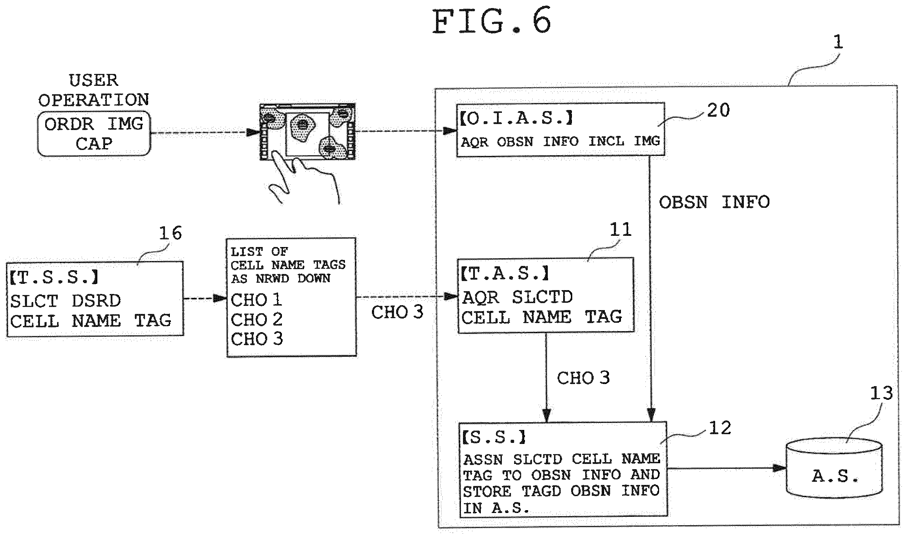

FIG. 6. is an explanatory diagram that schematically shows the flow of the procedure using the cell observation information processing system of FIG. 2, from acquisition of observation information; through selection of a search tag by a user from a list of search tags displayed on the presenting section; and up to storage of the observation information, to which the selected search tag is assigned, into the archive section.

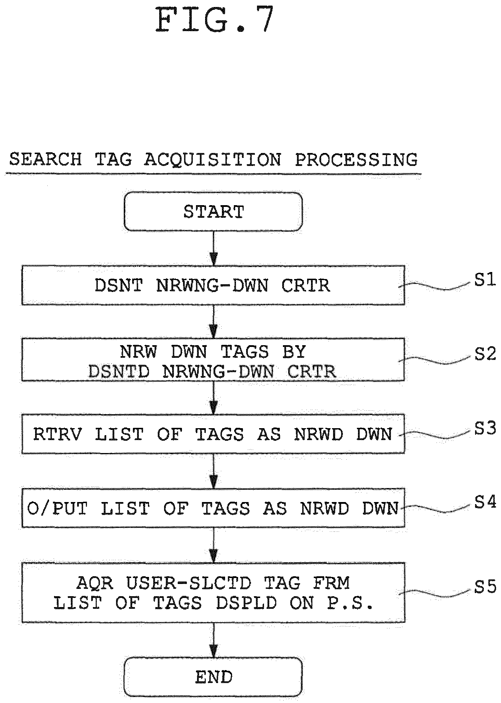

FIG. 7 is a flow chart that shows one example of the procedure using the cell observation information processing system of FIG. 2, from tag selection by a user; through narrowing down and retrieval of a list of search tags and display of the list of search tags; and up to storage of the observation information, to which the selected search tag is assigned, into the archive section.
FIG. 8. is an explanatory diagram that shows the configuration of the cell observation information processing system according to the second embodiment mode of the present invention.
FIGs. 9A-9B are explanatory diagrams that show data structure of tag acquisition history stored in the archive section in the cell observation information processing system of FIG. 8, where FIG. 9A shows one example and FIG. 9B shows another example.
FIG. 10 is an explanatory diagram that shows the configuration of the cell observation information processing system according to one modified example of FIG. 8.
FIG. 11 is an explanatory diagram that shows one example of data structure of tag acquisition history stored in the archive section in the cell observation information processing system of FIG. 10.
FIG. 12 is an explanatory diagram that shows the configuration of the cell observation information processing system according to the third embodiment mode of the present invention.
FIGs. 13A-13B are explanatory diagrams that show data structure with user-designated information stored in the archive section in the cell observation information processing system of FIG. 12, where FIG. 13A shows one example and FIG. 13B shows another example.
FIG. 14 is an explanatory diagram that shows the configuration of the cell observation information processing system according to one modified example of FIG. 12.
FIG. 15 is an explanatory diagram that shows one example of data structure with user-designated information stored in the archive section in the cell observation information processing system of FIG. 14.
FIG. 16 is an explanatory diagram that shows the configuration of the cell observation information processing system according to another modified example of FIG. 12.
FIG. 17 is an explanatory diagram that shows one example of data structure with user-designated information stored in the archive section in the cell observation information processing system of FIG. 16.
FIG. 18 is an explanatory diagram that shows the configuration of the cell observation information processing system according to still another modified example of FIG. 12.
FIG. 19 is an explanatory diagram that shows one example of data structure with user-designatedinformationstoredin the archive section in the cell observation information processing system of FIG. 18.
FIG. 20 is an explanatory diagram that shows the configuration of the cell observation information processing system according to one modified example of FIG. 2.
FIG. 21 is an explanatory diagram that shows the configuration of the cell observation information processing system according to another modified example of FIG. 2.
FIG. 22 is an explanatory diagram that shows the configuration of the cell observation information processing system according to still another modified example of FIG. 2.

### MODE FOR CARRYING OUT THE INVENTION

The embodiment modes of the present invention will be explained below. The embodiment modes explained below do not improperly limit the contents of the present invention recited in the claimed scope for a patent. In addition, not all of the configurations explained in reference to the embodiment modes below are indispensable configurations for the present invention.

A cell observation information processing system of the embodiment modes of the present invention includes: a tag acquiring section that acquires, as search tags for searching for observation information that has been acquired via an observation information acquiring section in response to an acquisition order at one time point to contain items carrying cell observation results such as a cell image, activity data indicating cell activity by a number of cells, a cell confluency, a morphology, a viability or so., and an observation title, at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; a storing section that assigns, as search tags, the tags acquired by the tag acquiring section to the observation information, which has been acquired in response to an acquisition order at each time point, and that stores search-tagged observation information, to which the search tags have been assigned, in an archive section; a retrieving section that retrieves from the archive section a list of tags as possible search tags; and an output section that outputs the list of tags retrieved by the retrieving section to a presenting section; wherein the retrieving section designates a narrowing-down criterion for narrowing down tags to be retrieved and retrieves the list of tags as narrowed down by the designated narrowing-down criterion, and the tag acquiring section acquires a tag selected by the user from the list of tags presented by the presenting section.

If the configuration is made so that the retrieving section designates a narrowing-down criterion for narrowing down tags to be retrieved and retrieves a list of tags as narrowed down by the designated narrowing-down criterion and so that the tag acquiring section acquires a tag selected by the user from the list of tags presented by the presenting section, as in the cell observation information processing system according to the embodiment modes of the present invention, the user can input in a short time, a tag to be assigned, as a search tag, to observation information only by a simple operation such as one button push for selecting a desired tag from a list of tags, even if the tag has a long string of letters, numerals or symbols.

Further, since the list of tags presented by the presenting section is a result of narrowing down to include only tags necessary for the user, the user can efficiently select a tag to be assigned to the observation information.

As a result, it is possible to save operation effort and time of the user taken to acquire tags to be assigned to observation information on cells. In particular, in a situation where operation for acquiring tags to be assigned to observation result of cells is made while the cells are being taken outside the incubator, the shortened time of user operation taken to acquire tags can moderate damage given to the cells, to assure accurateness of experiments.

As narrowing-down criteria, it is preferable to use user identifying information, information on tag acquisition history by the tag acquiring section, information on cell kind categorized into cell identifying information, information on passage/non-passage in cell maintenance work by user, etc.

In the embodiment modes of the present invention, the narrowing-down criterion for narrowing down tags to be retrieved by the retrieving section may be preliminarily determined by the user.

Other than the above-described effect, the embodiment modes of the present invention brings about the following subsidiary effects in accordance with the narrowing-down criterion to narrow down tags to be retrieved by the retrieving section or in accordance with the tags to be retrieved.

### (1) Function and effect of the configuration in which user (user oneself or acting worker) identifying information is designated as a narrowing-down criterion

In the cell observation information processing system of the embodiment modes of the present invention, the retrieving section preferably is configured to designate user identifying information as a narrowing-down criterion for narrowing down tags to be retrieved.

The user identifying information is identifying information for identifying the user who conducts cell observation. To be specific, this is identifying information for identifying the very person concerned who inputs cell observation result or an acting person who conducts cell observation or inputs cell observation result in place of the very person concerned.

In observation and experiment using cells, in accordance with the conventional custom, cell management tends to be conducted such that a particular limited user manages the cells throughout their entire lifecycle from birth to killing.

Under such observation and experiment circumstances in which each user independently conducts thorough management of cells, each user, in the situation of assigning search tags to acquired cell observation information, shows his or her particularity in notation of the search tags. Search tags with notations varying with particularities of individual users are convenient in use for the individual users who are accustomed to them.

Therefore, it is presumed that each user has a strong need to use search tags that were used in the past in assigning them to cell observation information under his or her own cell information management, and that search tags used under management of other users are unnecessary, or rather obstructive.

Thus, if the configuration is made so that the retrieving section designates user identifying information as a narrowing-down criterion for narrowing down tags to be retrieved as in the cell observation information processing system according to the embodiment modes of the present invention, it is possible for the user, in selecting from a list of tags presented by the presenting section a search tag to be assigned to the observation information he or she acquired, to have only highly usable tags, which have been used to be assigned to observation information under the very user's management, displayed on the presenting section while excluding tags that are less usable and not worth referring to, as they have been used to be assigned to observation information under other users' management.

This results in improved efficiency of user's selection of a tag to be assigned to observation information, to save operation effort and time of the user taken to acquire tags to be assigned to observation information on cells.

In particular, in a situation where operation for acquiring tags to be assigned to observation result of cells is made while the cells are being taken outside the incubator, the shortened time of user operation taken to acquire tags can moderate damage given to the cells, to assure accurateness of experiments.

### (1-1) Function and effect of the configuration in which user identifying information is designated as a narrowing-down criterion for narrowing down tags of cell identifying information

The cell observation information processing system of the embodiment modes of the present invention preferably is configured to narrow down tags of cell identifying information using the user identifying information.

The cell identifying information is identifying information for identifying cells that are an observation target. To be specific, it is identifying information that contains at least one of: cell name, cell kind, cell level information for identifying whether the cells are of the parent cell line to be cultured or of a subculture which have been separated from and cut out of the parent cell line, and passage number of the cells.

### (1-1-1) Function and effect of the configuration in which user identifying information is designated as a narrowing-down criterion for narrowing down cell name tags

A cell name is a name freely given by a user to cells used for experiment and research.

In some cases, of the cell identifying information, at least the notation of cell name can be freely determined by each user. In such a case, the notation of cell name is customized by each user; the notation of cell name tags given by a certain user tends to be inconvenient for other users.

Thus, if the configuration is made to narrow down cell name tags using the user identifying information as in the cell observation information processing system according to the embodiment modes of the present invention, it is possible for each user to have only tags convenient for use under his or her own management displayed in the form of a list of tags, while excluding cell name tags by other users, which are inconvenient for use, from the list of tags to be presented.

This results in improved efficiency of user's selection of a cell name tag to be assigned to observation information, to save operation effort and time of the user taken to acquire cell name tag to be assigned to observation information on cells.

In particular, in a situation where operation for acquiring a cell name tag to be assigned to observation result of cells is made while the cells are being taken outside the incubator, the shortened time of user operation taken to acquire the cell name tag can moderate damage given to the cells, to assure accurateness of experiments.

### (1-1-2) Function and effect of the configuration in which user identifying information is designated as a narrowing-down criterion for narrowing down cell kind tags

A cell kind is a specific category into which cells are classified.

There is large population of cell kinds, and the number of cell kinds is so large as several hundreds. On the other hand, cell kinds cultured as objects of research and experiment may vary with individual users. In such a case, as a search tag to be assigned to observation information that a certain user has acquired, cell kinds of other users are avoided, or unnecessary.

Therefore, in a case where a list of tags are presented for assigning a cell kind, as a search tag, to the acquired observation information, there is a strong need for each user to narrow down cell kinds to those under the user's own management.

Thus, if the configuration is made to narrow down cell kind tags using the user identifying information as in the cell observation information processing system according to the embodiment modes of the present invention, it is possible for each user to have only tags under his or her own management displayed in the form of a list of tags, while excluding tags of cell kinds under other users' management, which have poor usability, from the list of tags to be presented.

This results in improved efficiency of user's selection of a cell kind tag to be assigned to observation information, to save operation effort and time of the user taken to acquire a cell kind tag to be assigned to observation information on cells.

In particular, in a situation where operation for acquiring a cell kind tag to be assigned to observation result of cells is made while the cells are being taken outside the incubator, the shortened time of user operation taken to acquire the cell kind tag can moderate damage given to the cells, to assure accurateness of experiments.

### (2) Function and effect of the configuration in which information, on history of tag acquisition by the tag-acquiring section is designated as a narrowing-down criterion

In the cell observation information processing system of the embodiment modes of the present invention, the retrieving section preferably is configured to designate information on history of tag acquisition by the tag acquiring section as a narrowing-down criterion for narrowing down tags to be retrieved.

The information on history of tag acquisition includes: the latest date of tag acquisition by the tag acquiring section; the number of times of acquisition to date, the number of times of acquisition during a predetermined retrospective period of time from the present, etc.

In the cell observation information processing system of the embodiment modes of the present invention, the retrieving section preferably is configured to narrow down possibly retrievable tags to those having latest dates of acquisition later than a predetermined date or to those having values greater than a predetermined value with respect to the number of times of acquisition to date or the number of times of acquisition during a predetermined retrospective period of time from the present, by using the tag acquisition history information.

Under the circumstances in which each user cultures cells for research and experiment, a tag of cell kind or cell name that has been assigned to observation information on cells of the past, which are not currently cultured, seldom is used as a search tag to be assigned to observation information on currently cultured cells, but very often used is a tag that has been assigned to observation information on the cells currently cultured.

Thus, to present in the list of tags a currently inactive tag is meaningless, or rather obstructs tag selection by the user.

Thus, if the configuration is made so that the retrieving section designates information on history of tag acquisition by the tag acquiring section as a narrowing-down criterion for narrowing down tags to be retrieved as in the cell observation information processing system according to the embodiment modes of the present invention, it is possible to present, in the list of tags, only tags having being acquired at high frequency or many times or tags recently acquired, while excluding tags with poor usability such as tags having been acquired at low frequency or few times and tags of cells currently not being cultured, from the list of tags to be presented.

This results in improved efficiency of user's selection of a tag to be assigned to observation information, to save operation effort and time of the user taken to acquire a tag to be assigned to observation information on cells.

In particular, in a situation where operation for acquiring a tag to be assigned to observation result of cells is made while the cells are being taken outside the incubator, the shortened time of user operation taken to acquire the tag can moderate damage given to the cells, to assure accurateness of experiments.

### (2-1) Function and effect of the configuration in which the number of times of tag acquisition to date by the tag acquiring section is designated as a narrowing-down criterion

In the cell observation information processing system of the embodiment modes of the present invention, the retrieving section preferably is configured to designate the number of times of acquisition of tags to date by the acquiring section as a narrowing-down criterion for narrowing down tags to be retrieved.

For example, the number of cell kinds is so large as several hundreds. Therefore, in a case where cells of the same kind as the cells that have been once under management for observation and experiment should be observed and experimented on, presenting in the list of tags only cell kind tags that the user once has used for assignment to observation information is worthwhile.

Thus, if the configuration is made so that the retrieving section designates the number of times of tag acquisition by the tag acquiring section to date as a narrowing-down criterion for narrowing down tags to be retrieved as in the cell observation information processing system according to the embodiment modes of the present invention, for example in a case where cells of the same kind as the cells that have been once managed for observation and experiment should be managed for observation and experimentation, it is possible to exclude tags of cell kinds that have never been used from the list of tags to be presented.

### (2-2) Function and effect of the configuration in which the latest date of tag acquisition or the number of times of tag acquisition durin a predetermined retrospective period of time from the present by the tag acquiring section is designated as a narrowing-down criterion

In the cell observation information processing system of the embodiment modes of the present invention, the retrieving section preferably is configured to designate the latest date of tag acquisition or the number of times of tag acquisition during a predetermined retrospective period of time from the present by the tag acquiring section as a narrowing-down criterion for narrowing down tags to be retrieved.

A tag used as a search tag assigned to the observation information that has been under management for a period from a recent time point to the present time point is likely to be repeatedly used after the present time point also for a certain period. On the other hand, even if it has been used multiple times ever, a tag that is not used at the present time point is unlikely to be used from the present time point on.

Thus, if the configuration is made so that the retrieving section designates the latest date of tag acquisition or the number of times of tag acquisition in a predetermined retrospective period of time from the present by the tag acquiring section as a narrowing-down criterion for narrowing down tags to be retrieved as in the cell observation information processing system according to the embodiment modes of the present invention, it is possible to present only tags that have been used at high frequency during a period from a recent time point to the present time point in the form of a list of tags on the presenting section, while excluding, from the list of tags to be presented, tags that are inactive at the present time point, or that have low frequency of use.

Also, in the cell observation information processing system of the embodiment modes of the present invention, it preferably is configured so that information on history of tag acquisition by the tag acquiring section is designated as a narrowing-down criterion together with user identifying information.

In this case, it preferably is configured so that tags of cell identifying information, in particular, tags of cell name or cell kind are to be narrowed down.

This configuration makes it possible to present, in the form of a list of tags, only tags having high usability as search tags to be assigned to observation information at the present time point under the user's own management of cells, while excluding tags with lower usability at the present time point and tags under other users' management of cells from the list of tags.

This results in much improved efficiency of user's selection of a cell kind tag to be assigned to observation information, to further save operation effort and time of the user taken to acquire a cell kind tag to be assigned to observation information on cells.

In particular, in a situation where operation for acquiring a cell kind tag to be assigned to observation result of cells is made while the cells are being taken outside the incubator, the much shortened time of user operation taken to acquire the cell kind tag can further moderate damage given to the cells, to assure further accurateness of experiments.

### (3) Function and effect of the configuration in which a narrowing-down criterion is predetermined by a user

In the cell observation information processing system of the embodiment modes of the present invention, the narrowing-down criterion preferably is predetermined by the user.

### (3-1) Function and effect of the configuration in which a certain tag predetermined by a user is designated as a narrowing-down criterion

In the cell observation information processing system of the embodiment modes of the present invention, the narrowing-down criterion preferably is a certain tag predetermined by a user.

For example, users have a strong need to limit tags that are to be presented in the presenting section in the form of a list of tags, to those related to cells currently cultured. Further, for example, some user has a need to have certain tags presented on the presenting section as usable tags for future, even if the tags are not related to currently cultured cells. In this way, individual users have different favorite tags to be presented in the form of a list of tags.

Thus, if the configuration is made so that the narrowing-down criterion is a certain tag preliminarily determined by the user as in the cell observation information processing system according to the embodiment modes of the present invention, it is possible to directly retrieve and present, in the form of a list of tags, only favorite tags for each user, which reflect user's will for use, by narrowing down tags using the narrowing-down criterion preliminarily determined by the user oneself.

This results in improved efficiency of user's selection of a tag to be assigned to observation information, to save operation effort and time of the user taken to acquire a tag to be assigned to observation information on cells.

In particular, in a situation where operation for acquiring a tag to be assigned to observation result of cells is made while the cells are being taken outside the incubator, the shortened time of user operation taken to acquire the tag can moderate damage given to the cells, to assure accurateness of experiments.

As the predetermined tag, a cell name may be used, for example.

### (3-2) Function and effect of the configuration in which, out of a plurality of-narrowing-down criteria, a certain tag predetermined by a user is designated as a narrowing-down criterion

In the cell observation information processing system of the embodiment modes of the present invention, it is preferable that the retrieving section is configured to be able to designate a plurality of narrowing-down criteria and that, out of the plurality of narrowing-down criteria, a certain designated narrowing-down criterion has been preliminarily determined by a user.

As the plurality of narrowing-down criteria, applicable are categories tags belong to, such as user identifying information, information on user's work for cell maintenance, and information on history of tag acquisition by the tag acquiring section regarding tags that are possible objects of retrieval by the retrieving section.

For example, some user may wish to have a list of tags presented by narrowing-down methods using different narrowing-down criteria in accordance with cells under management, such as narrowing down by the user identifying information and narrowing down by the tag acquisition history.

Thus, if the configuration is made so that the retrieving section can designate a plurality of narrowing-down criteria and so that, out of a plurality of narrowing-down criteria, a designated narrowing-down criterion can be preliminarily determined by the user as in the cell observation information processing system of the embodiment modes of the present invention, it is possible to select a narrowing-down criterion that can be marked by the user as a favorite in accordance with the user's need, and to facilitate listing of only tags that are necessary for the user.

Further, if the configuration is made so that a user can preliminarily determine, as a plurality of narrowing-down criteria, categories tags belong to such as user identifying information, information on user's work for cell maintenance, and information on history of tag acquisition by the tag acquiring section regarding tags that are possible obj ects of retrieval by the retrieving section, the choice of narrowing-down criteria that can be marked by the user as favorites is widened in accordance with the user' s need, and to much facilitate listing of only tags necessary for the user.

### (4) Function and effect of the configuration in which the cell kind categorized in the cell identifying information can be designated as a narrowin down criterion and the object of narrowin down is cell name tags

In the cell observation information processing system of the embodiment modes of the resent invention, it is preferable that the narrowing-down criterion is information on cell kind, which is categorized in the cell identifying information, and that the retrieving section is configured to narrow down cell name tags categorized in the cell identifying information.

As a notation of cell name tags, a format made of combination of the cell kind (for example "CHO") and the identifying number for data management (for example "1", "2" etc.) is convenient, for a user who manages the cells concerned can recognize the cell kind of the object of observation only by the cell name as well as can distinguish them, in data management, from other data of the same kind of cells by the identifying number.

On the other hand, the number of cell kinds is so many as several hundreds, and thus if cell kinds are combined with identifying numbers for data management, the enormous number of cell names is likely.

Thus, if the configuration is made to narrow down cell names using a cell kind as a narrowing-down criterion as in the cell observation information processing system according to the embodiment modes of the present invention, in a case where the notation of cell name tags has the format of combination of the cell kind and the identifying number for data management, it is possible to exclude cell names of irrelevant cell kinds, which are likely to be tremendous, from the list of tags.

This results in improved efficiency of user's selection of a cell name tag to be assigned to observation information, to save operation effort and time of the user taken to acquire a cell name tag to be assigned to observation information on cells.

In particular, in a situation where operation for acquiring a cell name tag to be assigned to observation result of cells is made while the cells are being taken outside the incubator, the shortened time of user operation taken to acquire the cell name tag can moderate damage given to the cells, to assure accurateness of experiments.

### (5) Function and effect of the configuration in which information on passage/non-passage categorized in user's work for cell maintenance can be designated as a narrowing down criterion

In the cell observation information processing system of the embodiment modes of the present invention, the retrieving section preferably is configured to designate information on passage/non-passage categorized in user' s work for cell maintenance as a narrowing-down criterion for narrowing down tags to be retrieved.

Also, in the cell observation information processing system of the embodiment modes of the present invention, the retrieving section preferably is configured to narrow down tags of cell level information, which is categorized in the cell identifying information, for identifying whether the cells are of the parent line to be cultured or of a subculture which has been separated from and cut out of the parent cell line, using information on passage/non-passage categorized in user's work for cell maintenance.

In general, cells to be used for experiment and research are separated from the living body and cultured upon being planted in a culture medium in a certain culture container, to be proliferated. When the cells are proliferated to a predetermined confluency or number, a part of the cells in the culture container is transplanted to a culture medium in another culture container. This process is called subculture (passage of culture). The cells for experiment and research separated from the parent cell line are called subculture.

At the date when a user conducts passage, since the subculture, just after separation and cutting out, contains too small a number of cells to make it possible to acquire observation information on them or to assign a search tag, acquisition of observation information and assignment of search tags are made only for the parent cell line. Regarding the subculture, a few days after the date of separation and cutting out, when they are proliferated to have so large a number of cells as to allow observation information on them to be acquired, acquisition of observation information and assignment of search tags are made.

While the subculture is used for an experiment after separation and cutting out, cell culturing of the parent cell line is continued, and the work for subculture, or cutting out and transplanting of a part of the cells to a culture medium in another culture container, is repeated each time the cells are proliferated to reach a predetermined number of cells or cell confluency.

In this way, for the same kind of cells, at the date when a user conducts passage of culture, the cell level for which acquisition of observation information and assignment of search tags are made is limited to the parent cell line. In addition, at the date when the passage of culture is made, even if observation information and search tags for a subculture exist in the archive section, these observation information and search tags belong to a subculture of an earlier generation separated and cut out from the parent cell line before the current passage. Therefore, search tags assigned to observation information of a subculture stored in the archive section at the date of passage do not have usability as tags to be assigned to observation information of the parent cell line and thus, if presented in the list of tags, rather obstruct tag selection by the user.

Thus, if the configuration is made so that the retrieving section designates the information on passage/non-passage categorized in user's work for cell maintenance as a narrowing-down criterion for narrowing down tags to be retrieved as in the cell observation information processing system according to the embodiment modes of the present invention, it is possible, for example by conducting, at the date when user makes passage of culture for a plurality of kinds of cells all together, narrowing down to search tags assigned to observation information on cells that previously underwent passage, to exclude, from the list of tags to be presented, search tags assigned to observation information on unnecessary subculture or the parent cell line at the non-passage timing.

Also, the cell observation information processing system of the embodiment modes of the present invention preferably is further provided with a presenting section for presenting for a user a list of tags retrieved by the retrieving section.

If the cell observation information processing system is provided with a presenting section in this way, a user can acquire, in a short time without trouble, a tag to be assigned to observation information, as a search tag, only by selecting a desired tag from the list of tags displayed on the presenting section provided in the cell observation information processing system, without using another presenting section independent of the cell observation information processing system.

Hereinafter, an explanation is made on the embodiment modes of the present invention in reference to the drawings.

### First embodiment mode

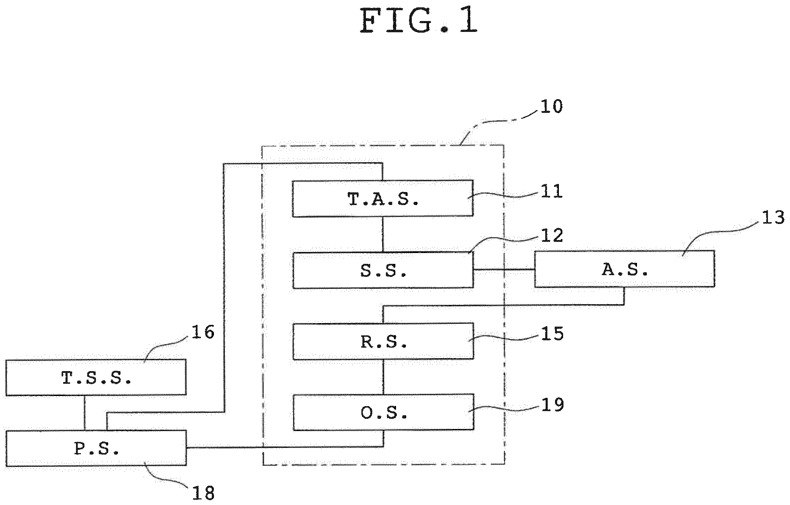

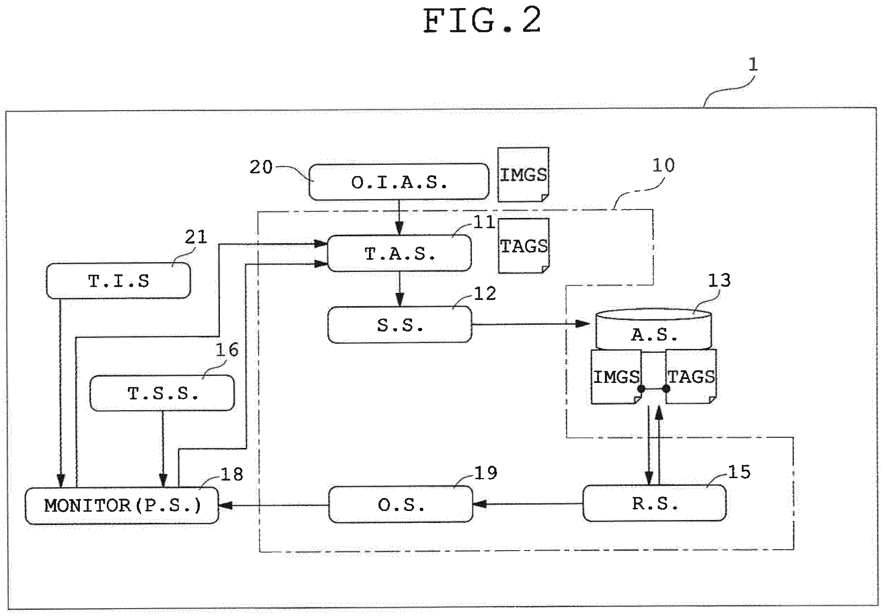

FIG. 1 is a block diagram that schematically shows the basic configuration of the cell observation information processing system according to the present invention. FIG. 2 is an explanatory diagram that shows the configuration of the cell observation information processing system according to the first embodiment mode of the present invention.

The cell observation information processing system 10 of the first embodiment mode includes, as shown in FIG. 1 and FIG. 2, a tag acquiring section 11, a storing section 12, a retrieving section 15 and an output section 19. In FIG. 1 and FIG. 2, the reference numeral 1 denotes a cell observation information processing installation, the reference numeral 13 denotes an archive section, the reference numeral 18 denotes a monitor as a presenting section, the reference numeral 16 denotes a tag selecting section, the reference numeral 20 denotes an observation information acquiring section, and the reference numeral 21 denotes a tag input section. In FIG. 2, the cell observation information processing system 10 is configured as a stand-alone system with the tag acquiring section 11, the storing section 12, the retrieving section 15 and the output section 19 being provided in the single installation 1 together with the archive section 13, the presenting section 18, the tag selecting section 16, the tag input section 21 and the observation information acquiring section 20. The cell observation information processing installation 1 is configured, for example of a microscope apparatus provided with an image capture apparatus and a computer provided with a database.

The tag acquiring section 11 acquires, as search tags for searching for observation information that has been acquired via an observation information acquiring section 20 in response to an acquisition order at one time point to contain items carrying cell observation results such as a cell image, activity data indicating cell activity by a number of cells, a cell confluency, a morphology, a viability or so., and an observation title, at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image.

To be specific, the tag acquiring section 11 is configured to acquire, as search tags, for example a user ID selected and inputted by a user in a user ID input screen (not shown in the drawings) provided in the cell observation information processing installation 1, and cell level information ("parent cell line" or "subculture"), a cell name, a passage number etc. inputted by the user in input fields of a search tag input screen (not shown in the drawings) in the tag input section 21.

Other than those, the tag acquiring section 11 acquires measurement date/time automatically generated by the system such as image capture date/time having been recorded as a log in a cell image in each observation information acquired via the observation information acquiring section 20, as date/time information for identifying the date/time when the observation information was acquired. Further, the tag acquiring section 11 is configured to be capable of acquiring an image tag from the cell image in each observation information acquired via the observation information acquiring section 20.

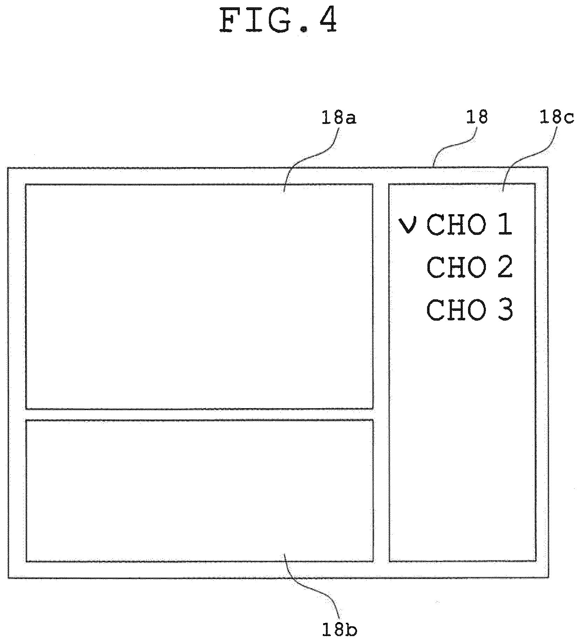

According to this embodiment mode, the tag acquiring section 11 is further configured to be capable of acquiring, for example a tag selected by the user via the tag selecting section 16, which will be described later, from a list of tags displayed in a pull-down menu format on the display surface 18c of the presenting section 18 shown in FIG. 4, and tags manually inputted by the user with keys of characters, numerals and symbols via the tag input section 21, which will be described later, into a tag input screen (not shown in the drawings) displayed on the presenting section 18.

The storing section 12 is configured to assign, as search tags for searching for observation information, the tags acquired by the tag acquiring section 11 to each observation information acquired by the observation information acquiring section 20 in response to an acquisition order at each time point, and to store the search-tagged observation information, to which the search tags are assigned, in the archive section 13.

It is noted that the information on user's work for cell maintenance are identifying information containing at least one of work detail, dilution ratio, container's type or size, and container address where the cells are contained. The dilution rate is defined as an amount of thinning in a situation where cells cultured in a container are thinned for passage or experiment.

The image tag is a tag for indicating a cell image, to be specific, an image displayable in a small size in an image selection and input screen (not shown in the drawings) presented on the presenting section 12 or information containing an image and its corresponding name integrally combined together.

The activity data variation information is expressed, for example by an amount of variation (that is, a rate of variation) between respective activity data (for example, cell confluency, number of cells or viability) at a plurality of time points, and can be shown, for example by a slope of a plotted line in a graph.

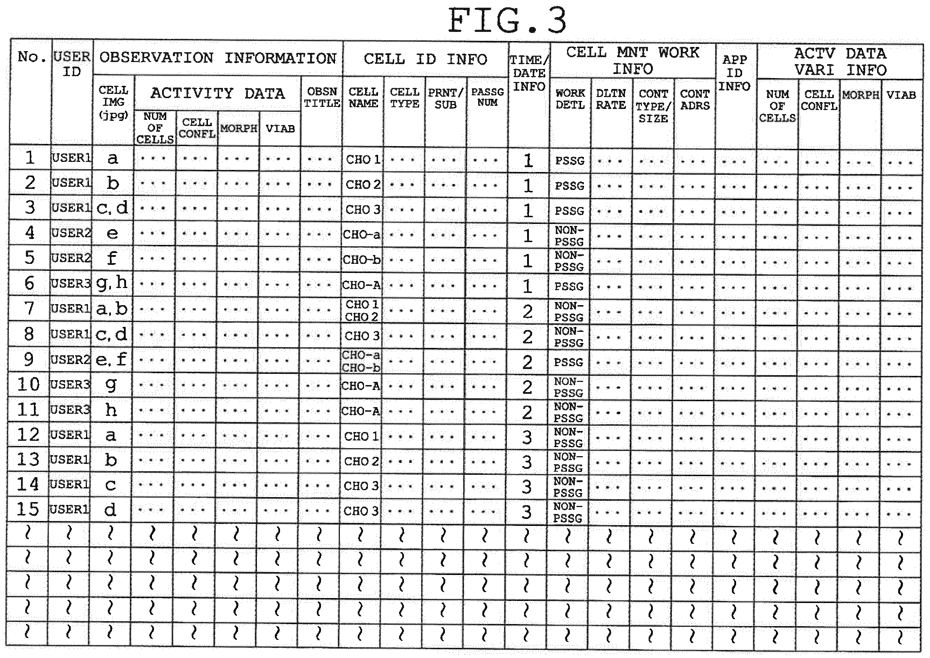

The archive section 13 has, as shown in FIG. 3, one or more records of the search-tagged observation information. Each record is created in response to image capture at one time point of cell images, where observation information corresponding to the image capture at one time of the cell images is related with search tags for retrieving the observation information.

While the user ID is used as the user identifying information in this embodiment mode for convenience' sake, the user identifying information is not necessarily limited to the user ID; any letters or symbols that can distinguish a certain user from other users, such as a name or nickname of the user, may work.

The retrieving section 15 is configured to designate a narrowing-down criterion for narrowing down tags as possible search tags to be retrieved, and to retrieve, from the archive section 13, a list of tags as possible search tags resulted from the narrowing down with the designated narrowing-down criterion.

The output section 19 is configured to output the list of tags retrieved by the retrieving section 15 to the presenting section 18.

The presenting section 18 has a display field 18c that displays the list of tags outputted by the output section 19 for example in the pull-down menu format as shown in FIG. 4. In FIG. 4, the reference numeral 18a denotes a display field for cell image, and the reference numeral 18b denotes a display field for activity data.

The tag selecting section 16 is configured to allow a user to select a desired tag from the list of tags displayed in the pull-down menu format in the display field 18c of the presenting section 18.

The tag input section 21 is configured to allow a user to manually input desired tags using keys of letters, numerals and symbols into the tag input screen (not shown in the drawings) displayed on the presenting section 18. The tag input section 21 is used in situations where a user desires to create a setting with respect to tags that are not included in the list of tags retrieved by the retrieving section 15 and displayed on the display field 18c of the presenting section 18, such as the situation where the user is to make initial setting of a tag as a search tag to be assigned to observation information.

The observation information acquiring section 20 is configured to acquire observation information in response to an acquisition order at one time point. To be specific, the observation information acquiring section 20 is actuated when a user inputs an observation name and pushes an order button for observation information acquisition on an image-capture order screen (not shown in the drawings), makes an image capture apparatus (not shown in the drawings) provided in the installation 1 image-capture a specimen containing cells placed at an observation position, detectsactivity data that show cell activity in terms of cell confluency, morphology, viability or so by subjecting a cell image as captured to image processing and analysis processing via an image processing section and an image analyzing section (not shown in the drawings), and temporally stores, in a storage area (not shown in the drawings) in the installation 1, one set of observation information including the cell image, the activity data and the observation name.

The cell observation information processing installation 1 is provided with a system log-in screen not shown in the drawings. If a user inputs a log-in ID and a password of the system in the system log-in screen, a user-ID input screen not shown in the drawings is displayed. In the user-ID input screen, if the user inputs, by selection, his or her user ID as the user identifying information, the user ID is temporally stored in the storage area (not shown in the drawings) in the installation 1 and an image-capture order screen (not shown in the drawings) is displayed so that the user can perform operation for image-capture order. The user-ID input screen not shown in the drawings may be configured to function as a system log-in screen also by letting a user perform input operation for a log-in ID and a password of the system together with input operation by selection of a user ID.

InreferencetoFIGs. 5-7, an explanation is made on the processing procedure from selection of a search tag by a user from a list of search tags displayed on the presenting section 18 to storage, in the archive section 13, of observation information to which the selected search tag is assigned using the cell observation information processing system according to the first embodiment mode thus configured.

The procedure of condition management of cells cultured by users is divided into the stage of storing observation information on the cells under culture into the archive section 13 and the stage of searching and retrieving desired observation information on cells from the archive section 13 and checking conditions of the cells. Selection of a search tag by a user from the list of search tags displayed on the presenting section 18 and storage of the observation information to which the selected search tag is assigned are made at the stage of storing observation information on the cells under culture into the archive section 13.

First, the processing procedure from narrowing down and retrieval of the list of search tags to display of the list of search tags as narrowed down and retrieved is explained.

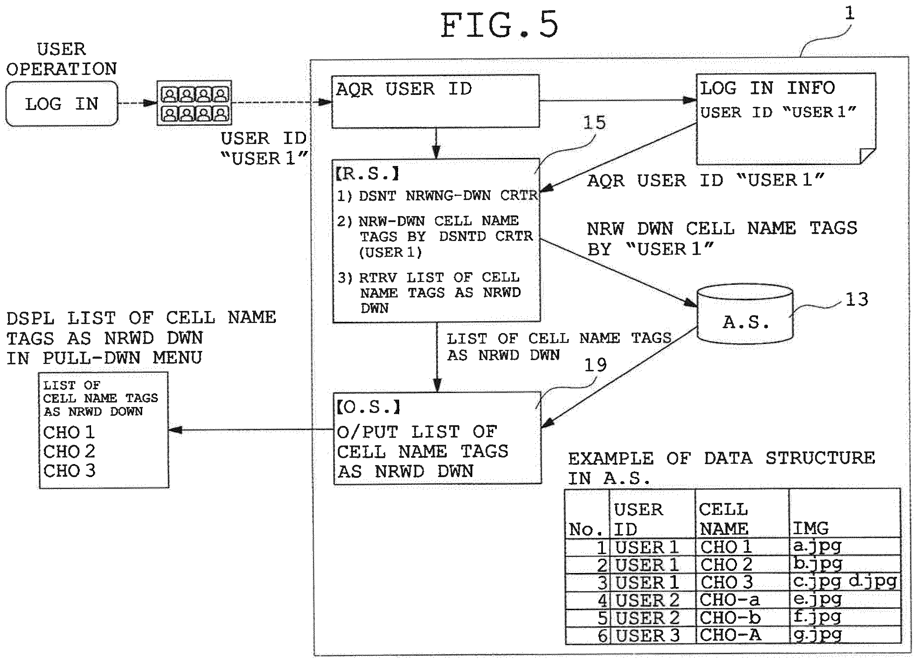

The user inputs a log-in ID and a password of the system in the system log-in screen (not shown in the drawings). Then, the user ID input screen not shown in the drawings is displayed. Then, the user inputs his or her own user ID (in the example of FIG. 5, "USER 1") by selection. Consequently, the user ID is temporally stored in the storage area in the installation 1 and an image-capture order screen (not shown in the drawings) is displayed, to allow the user to perform operation for image-capture order.

Initially, the retrieving section 15 designates the user ID ("USER 1") temporally stored in the storage area of the installation 1 as a narrowing down criterion (Step S1).

Then, the retrieving section 15 narrows down cell name tags stored in the archive section 13 as being assigned to observation information as search tags, to the cell name tags coexisting with the user ID ("USER 1") as the narrowing down criterion (in the example of FIG. 3, "CHO 1", "CHO 2" and "CHO 3")(Step S2).

Then, the retrieving section 15 retrieves a list of tags as narrowed down (Step S3).

Then, the output section 19 outputs the list of cell name tags retrieved by the retrieving section 15 to the presenting section 18 (Step S4).

Thereby, the list of cell name tags is displayed on the presenting section 18 in the pull-down menu format, to allow the user to select a search tag to be assigned to observation information via the tag selecting section 21.

Next, the processing procedure from selection of a search tag by the user from the list of search tags displayed on the presenting section 18 to storage into the archive section 13 of the observation information to which the selected search tag is assigned is explained.

As stated above, by inputting a log-in ID and a password of the system in the system log-in screen (not shown in the drawings) and then inputting his or her user ID by selection in the user ID input screen (not shown in the drawings) then displayed, the user can perform operation for image-capture order upon the image-capture order screen (not shown in the drawings) being displayed.

The user inputs an observation name in the image-capture order screen (not shown in the drawings) and pushes an order button for ordering acquisition of observation information. Then, the observation information acquiring section 20 makes the image capture apparatus (not shown in the drawings) image-capture a specimen containing cells placed at the observation position, detects activity data that show cell activity in terms of number of cells, cell confluency, morphology, viability or so, for example by subjecting a cell image as captured to image processing and analysis processing via the image processing section and the image analyzing section (not shown in the drawings), and temporally stores, in the storage area (not shown in the drawings) in the installation 1, one set of observation information including the cell image, the activity data and the observation name. In this way, the observation information at one time point is acquired. It is noted that acquisition of observation information can be repeated a plurality of times for different acquisition time points.

Upon completion of input and observation information acquisition order by the user from the image-capture order screen and acquisition of observation information by the observation information acquiring section 20, the user selects a desired cell name tag (here, supposed "CHO 3" is selected) from the list of cell name tags ("CHO 1", "CHO 2", "CHO 3"), which are the result of narrowing down with the user ID ("USER 1") as the narrowing-down criterion and are displayed in the pull-down menu format, via the tag selecting section 21. When the selection is completed, the tag acquiring section 11 acquires the cell name tag "CHO 3" as a search tag to be assigned to the observation information (Step S5). In this example, the configuration is made so that, after a plurality of times of repetition of observation information acquisition by the observation information acquiring section 20, the presenting section 18 is switched to the mode for selection of a desired tag from the list of cell name tags displayed in the pull-down menu format and acquisition of the search tag by the tag acquiring section 11. However, the configuration may be made so that the presenting section 18 is switched each time after acquisition of the observation information by the observation information acquiring section 20, to the mode for selection of a desired tag from the list of cell name tags displayed in the pull-down menu format and acquisition of the search tag by the tag acquiring section 11. Regarding timing of assigning the search tags, the configuration may be made so that the presenting section 18 is switched to the mode for selection of a desired tag from the list of cell name tags displayed in the pull-down menu format and acquisition of the search tag by the tag acquiring section 11 in real time after acquisition of observation information by the observation information acquiring section 20, or so that the presenting section 18 is switched to the mode for selection of a desired tag from the list of cell name tags displayed in the pull-down menu format and acquisition of the search tag by the tag acquiring section 11 a certain time after acquisition of observation information by the observation information acquiring section 20.

After that, the storing section 12 assigns the cell name tag ("CHO 1"), which has been inputted by the user by selection from the list of cell name tags displayed in the pull-down menu format and has been acquired by the tag acquiring section 11, as a search tag, to each set of observation information acquired in response to the acquisition order at each time point, and stores the search-tagged observation information, to which the search tag is assigned, into the archive section 13.

Upon completion of assignment of the search tag to observation information of every time point and storage of the search-tagged observation information into the archive section 13, the procedure at the stage of storing observation information on cells under culture into the archive section 13 finishes.

According to the cell observation information processing system 10 of the first embodiment mode, since the configuration is made so that the retrieving section 15 designates a narrowing-down criterion for narrowing down tags to be retrieved and retrieves a list of tags as narrowed down by the designated narrowing-down criterion and so that the tag acquiring section 11 acquires a tag selected by the user from the list of tags presented by the presenting section 18, the user can input, in a short time, a tag to be assigned, as a search tag, to observation information only by a simple operation such as one button push for selecting a desired tag from the list of tags, even if the tag has a long string of letters, numerals or symbols.

Further, since the list of tags presented by the presenting section 18 is a result of narrowing down to include only tags necessary for the user, the user can efficiently select a tag, as a search tag to be assigned to the observation information.

Therefore, according to the cell observation information processing system 10 of the first embodiment mode, it is possible to save operation effort and time of the user taken to acquire tags to be assigned to observation information on cells. In particular, in a situation where operation for acquiring a tag to be assigned to observation result of cells is made while the cells are being taken outside the incubator, the shortened time of user operation taken to acquire the tag can moderate damage given to the cells, to assure accurateness of experiments.

In the above-described embodiment mode, for convenience's sake, it is supposed that the narrowing-down criterion designated by the retrieving section 15 is a user ID and that tags to be narrowed down and retrieved are cell name tags. However, the narrowing-down criterion designated by the retrieving section 15 or tags to be narrowed down and retrieved in the form of a list are not limited to this supposition.

For example, when the retrieving section 15 designates a user ID as a narrowing-down criterion, tags to be narrowed down and retrieved in the form of a list at least may belong to any of the cell kind, the cell level information for identifying parent cell line/subculture, and the passage number, which are subcategories of the cell identifying information as shown in FIG. 3.

Alternatively, for example, the narrowing-down criterion designated by the retrieving section 15 may be set to information on passage/non-passage categorized in user's work for cell maintenance (in the example of FIG. 3, work detail categorized in cell maintenance work information). In this case, tags to be narrowed down and retrieved in the form of a list may belong to cell level information for identifying parent cell line/subculture in cell identifying information shown in FIG. 3 example.

Alternatively, for example, the narrowing-down criterion designated by the retrieving section 15 may be set to information on cell kind categorized in cell identifying information as shown in FIG. 3 example, and tags to be narrowed down and retrieved in the form of a list may belong to cell name categorized in cell identifying information.

While this embodiment mode as described above is configured so that a user ID as the narrowing down criterion designated by the retrieving section is automatically set by user operations such as inputting a log-in ID and a password of the system in the log-in screen (not shown in the drawings) of the system and inputting the user's own user ID by selection on the user-ID input screen (not shown in the drawings), the embodiment modes of the present invention are not limited to this configuration. For example, the configuration may be made so that a user can freely set or change the narrowing-down criterion by inputting a desired narrowing-down criterion upon a narrowing-down criterion setting/change input screen (not shown in the drawings) being provided or by selecting a desired narrowing-down criterion upon a narrowing-down criterion setting/change selecting screen (not shown in the drawings) being provided. Alternatively, for example, a predetermined narrowing-down criterion may be provided as a default setting in a software that operates the computer of the cell observation information processing system of the embodiment modes of the present invention.

Other than that, the cell observation information processing installation 1 is provided with a search criterion designating screen (not shown in the drawings), which makes it possible to designate, as a search criterion, information containing, among the search tags assigned to the search-tagged observation information, at least one of a user ID as user identifying information, a cell name as cell identifying information, cell level information ("parent cell line" or "subculture"), work detail as information on user's work for cell maintenance, an apparatus ID as apparatus identifying information, and an activity rate as activity data variation information.

The cell observation information processing system 10 has a search criterion acquiring section and an observation data retrieving section (not shown in the drawings) also. The search criterion acquiring section acquires, as a search criterion, for example a combination of activity data and date/time information as added to the information inputted by the user in the search criterion designating screen. The observation data retrieving section searches through the archive section 13 using the search criterion acquired by the search criterion acquiring section, and retrieves data containing search-tagged observation information to which search tags matched with the search criterion have been assigned. The data containing search-tagged observation information retrieved by the search criterion retrieving section is displayed on a display surface (not shown in the drawings) in a display mode that depends on the search criterion acquired by the search criterion acquiring section.

Through these steps of inputting a search criterion by the user on the search criterion designating screen, acquiring the search criterion by the search criterion acquiring section, retrieving data containing search-tagged observation information by the observation data retrieving section and displaying on the display surface, it is possible to conduct the procedure at the stage of searching and retrieving desired observation data on cells from the archive section and checking the cell conditions.

### Second embodiment mode

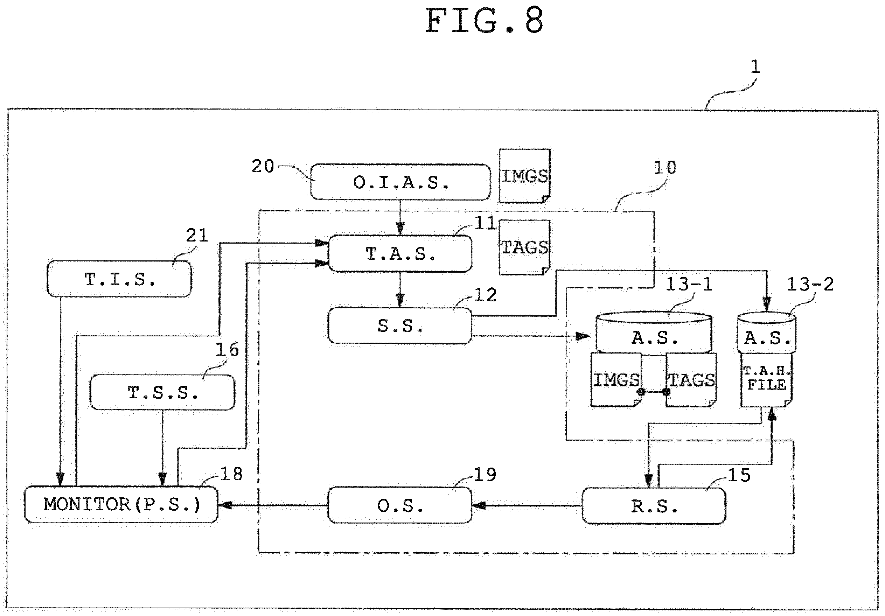

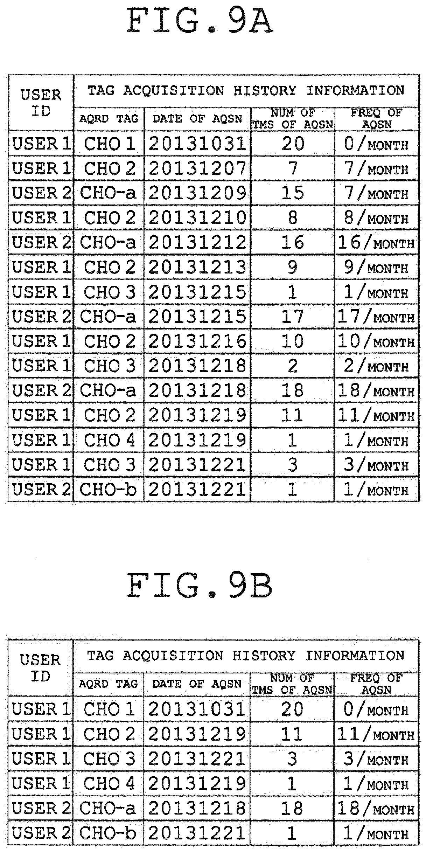

FIG. 8. is an explanatory diagram that shows the configuration of the cell observation information processing system according to the second embodiment mode of the present invention. FIGs. 9A-9B are explanatory diagrams that show data structure of tag acquisition history stored in the archive section in the cell observation information processing system of FIG. 8, where FIG. 9A shows one example and FIG. 9B shows another example.

In the cell observation information processing system 10 of the second embodiment mode, the storing section 12 is configured to store search-tagged observation information as shown in FIG. 3 into a first archive section 13-1 as well as to store information on history of tag acquisition by the tag acquiring section 11 upon relating it to at least one of the user identifying information and the cell identifying information, into a second archive section 13-2, which is a file different from the first archive section 13-1. The archive section is provided with the first archive section 13-1 and the second archive section 13-2. The first archive section 13-1 is configured as a database file. The second archive section 13-2 is configured, for example as a log-in file of the system.

The information on tag acquisition history for example contains, as shown in FIGs. 9A and 9B, tag acquired by the tag acquiring section 11, acquisition date of tag, total number of times of acquisition to date (time point of tag acquisition), and the number of times of acquisition during a predetermined retrospective period of time from the present (time point of tag acquisition).

In the second archive section 13-2, for example as shown in FIG. 9A, there is stored information on tag acquisition history related to search-tagged observation information for each record. Alternatively, in the second archive section 13-2, for example as shown in FIG. 9B, there is stored updated information on tag acquisition history in which the acquisition date, the total number of times of acquisition to date (time point of tag acquisition) and the number of times of acquisition during a predetermined retrospective period of time from the present (time point of tag acquisition) are, as being directed to the identical user ID and the acquired tag (in this example, cell name), updated at each time point when search-tagged observation information is stored into the first archive section 13-1.

In FIG. 9A and FIG. 9B, the total number of number of times of acquisition to date (time point of tag acquisition) is written as "NUM OF AQSN" (number of acquisition), and the number of times of acquisition during a predetermined retrospective period of time from the present (time point of tag acquisition) is written as "FREQ OFAQSN" (frequency of acquisition) for convenience's sake. In FIG. 9A and FIG. 9B, "FREQ OF AQSN" (frequency of acquisition) is represented by the number of times of acquisition during one retrospective month from the present (time point of tag acquisition). In the example of FIG. 9A, instead of providing the number of times of acquisition during a predetermined retrospective period of time from the present (time point of tag acquisition)as a part of the tag acquisition history, it may be configured so that the retrieving section 15, before designating a narrowing-down criterion for retrieving a list of tags, calculates, for example, the number of times of tag acquisition during a period designated by a user in a period-designating screen (not shown in the drawings), using the date of tag acquisition and the total number of times of acquisition to date (time point of tag acquisition) stored in the second archive section 13-2.

The retrieving section 15 is configured to retrieve a list of certain tags by performing narrowing down using the information on tag acquisition history stored in the second archive section 13-2. In this embodiment mode, the retrieving section 15 is configured to perform narrowing down, using the information on tag acquisition history as relating it to the user ID.

Narrowing down by the retrieving section 15 using the information on tag acquisition history is performed to narrow down tags with the following criteria, for example.
(Criterion 1) tags with a latest date of acquisition later than a predetermined date
(Criterion 2) tags with a total number of times of acquisition to date greater than a predetermined value
(Criterion 3) tags with a number of times of acquisition during a certain retrospective period from the present greater than a predetermined value

For example, let us suppose that the present date is December 23, 2013. In the case of retrieving a list of cell name tags under management of the user having the user ID "USER 1" , which are possible search tags, with the above criterion 1, if the threshold of the latest date of acquisition is set to be December 16, 2013, the retrieving section 15 retrieves, in the form of a list of tags, "CHO 2" and "CHO 4", which have the latest date of acquisition of December 19, 2013, and "CHO 4", which has the latest date of acquisition of December 21, 2013.

Alternatively, for example, in the case of retrieving a list of cell name tags as possible search tags with the above criterion 2, if the threshold of the total number of times of acquisition is set to be 10 times, the retrieving section 15 retrieves, in the form of a list of tags, "CHO 1" and "CHO 2", which have the total number of times of acquisition of 20 times and 11 times, respectively.

Alternatively, for example, in the case of retrieving a list of cell name tags as possible search tags with the above criterion 3, if the narrowing-down criterion is set to be tags with 3 or more times of acquisition during one retrospective month from the present (that is, from November 23, 2013 to December 23, 2013), the retrieving section 15 retrieves, in the form of a list of tags, "CHO 2" and "CHO 3", which have the number of times of tag acquisition during the past one month from the present of 11 times and 3 times, respectively.

At the timing of storing search-tagged observation information into the first archive section 13-1, the storing section 12 stores, into the second archive section 13-2, information on tag acquisition history upon relating it with user identifying information, for example in such a manner as shown in FIG. 9A where the information on tag acquisition history is related to search-tagged observation information for each record or in such a manner as shown in FIG. 9B where the acquisition date, the total number of times of acquisition to date (time point of tag acquisition), and the number of times of acquisition during a predetermined retrospective period of time from the present (time point of tag acquisition) are updated.

Other configurations are substantially the same as the cell observation information processing system 10 of the first embodiment mode.

According to the cell observation information processing system 10 of the second embodiment mode, since the retrieving section 15 is configured to designate, as a narrowing-down criterion for narrowing down tags to be retrieved, information on history of tag acquisition by the tag acquiring section 11, it is possible to present, in the list of tags, only tags having being acquired at high frequency or many times or tags recently acquired, while excluding tags with poor usability such as tags having been acquired at low frequency or few times and tags of cells currently not being cultured, from the list of tags to be presented.

In the cell observation information processing system 10 of FIG. 8, the storing section 12 is configured to store the information on history of tag acquisition by the tag acquiring section 11 into the second archive section 13-2, which is a file different from the first archive section 13-1 as a file to store the search-tagged observation information. However, the cell observation information processing system 10 of the second embodiment mode may be configured so that the storing section 12 stores the information on history of tag acquisition by the tag acquiring section 11 into an archive section in the same file as the file to store the search-tagged observation information.

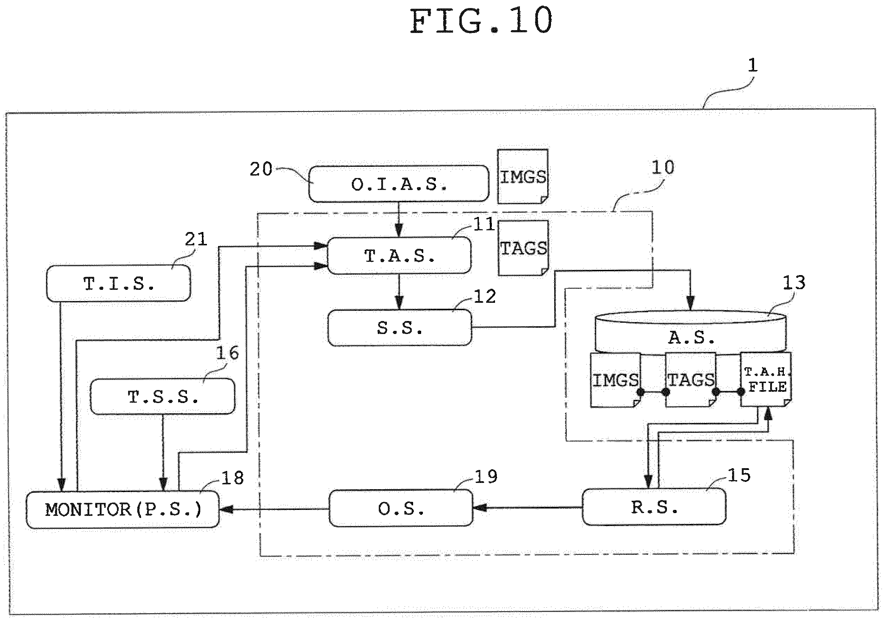

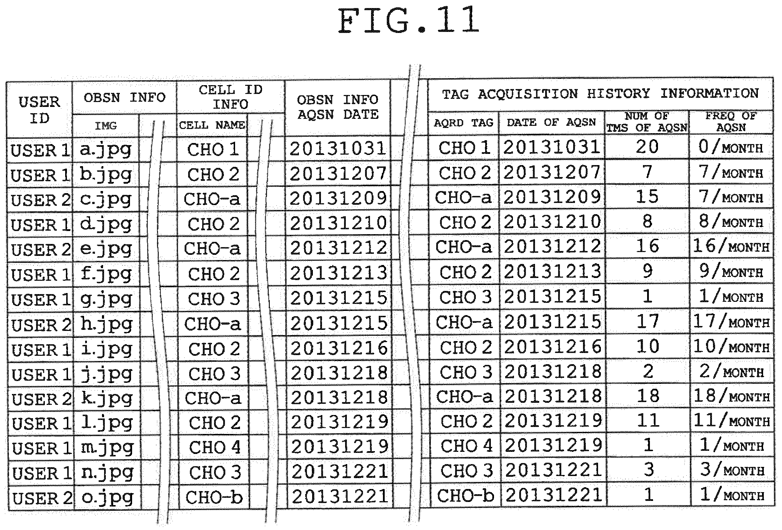

FIG. 10 is an explanatory diagram that shows the configuration of the cell observation information processing system according to one modified example of FIG. 8. FIG. 11 is an explanatory diagram that shows one example of data structure of tag acquisition history stored in the archive section in the cell observation information processing system of FIG. 10.

In the cell observation information processing system 10 of this modified example, the storing section 12 is configured to store, into the archive section 13, information on tag acquisition history upon assigning it to search-tagged observation information. The archive section 13 provides the information on tag acquisition history, which relates to the search-tagged observation information, as data items building a record, as shown in FIG. 11.

Other configurations and functions and effects are substantially the same as in the case of the cell observation information processing system 10 of FIG. 8.

### Third embodiment mode

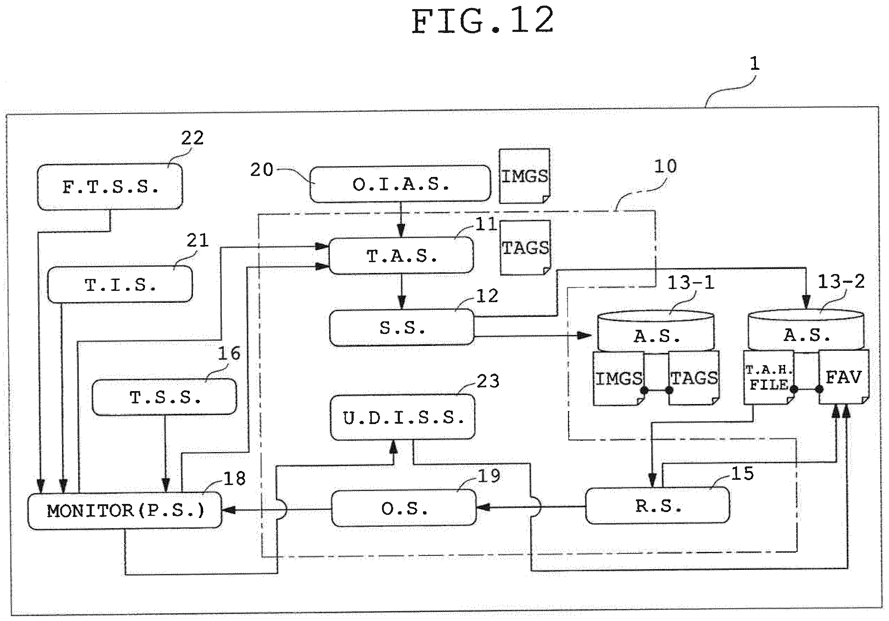

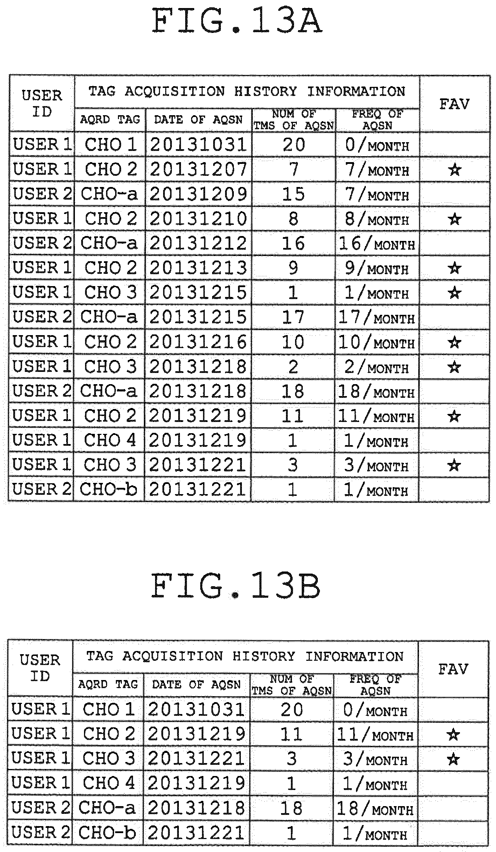

FIG. 12 is an explanatory diagram that shows the configuration of the cell observation information processing system according to the third embodiment mode of the present invention. FIGs. 13A-13B are explanatory diagrams that show data structure with user-designated information stored in the archive section in the cell observation information processing system of FIG. 12, where FIG. 13A shows one example and FIG. 13B shows another example.

In the cell observation information processing system 10 of the third embodiment mode, a user-designated-information storing section 23 is provided, and the narrowing-down criterion designated by the retrieving section 15 has been preliminarily determined by the user.

The user-designated-information storing section 23 is configured to store, into the archive section, user-designated information directed to a certain tag designated via a favorite-tag setting section 22, upon relating it to at least one of the cell identifying information and the user identifying information.

The favorite-tag setting section 22 is configured to make it possible to designate a certain tag that the user desires to make the retrieving section 15 designate as a narrowing-down criterion, via an operation by selection, from a list of tags (not shown in the drawings) in a tag designating screen displayed for example in the pull-down menu format on the presenting section 18 by a certain operation.

The cell observation information processing installation 1 of FIG. 12 is configured of the cell observation information processing installation 1 shown in FIG. 8 further provided with the user-designated-information storing section 23 inside the cell observation information processing system 10 and the favorite-tag setting section 22 outside the cell observation information processing system 10.

In the cell observation information processing system 10 of FIG. 12, the user-designated-information storing section 23 is configured to store user-designated information directed to a certain tag designated via the favorite-tag setting section 22, upon relating it to the user ID and assigning it to the information on tag acquisition history stored in the second archive section 13-2.

For example, in the case where the cell names "CHO 2" and "CHO 3" are designated via the favorite-tag setting section 22 as tags that the user having the user ID "USER 1" desires tomake the retrieving section 15 designate as a narrowing-down criterion, the user-designated-information storing section 23 relates a mark formed of a letter, symbol or figure (for example, pentagram) indicating user's favorite, as user-designated information directed to the cell names "CHO 2" and "CHO 3", to the user ID "USER 1". Then, as shown in FIG. 13A and FIG. 13B, the mark is put in the column of favorite-information setting in each of the records having the user ID "user 1" and the cell name "CHO 2" and the records having the user ID "USER 1" and the cell name "CHO 3", out of records of information on acquisition history of individual tags stored in the archive section 13-2, to thereby update these records.

Also, in the cell observation information processing system 10 of FIG. 12, the retrieving section 15 narrows down the records of information on tag acquisition history stored in the archive section 13-2 to those given the mark (pentagram) indicating the favorite by the user-designated information storing section 23, and retrieves the cell names "CHO 2" and "CHO 3" in the form of a list of tags.

The favorite-tag setting section 22 is configured also to allow cancellation of designation with respect to a tag for which the user desires cancellation of designation, via an operation by selection, from a list (not shown in the drawings) of tags already designated as "tags that the user desires to make the retrieving section 15 designate as a narrowing-down criterion" in a tag designation cancelling screen displayed for example in the pull-down menu format on the presenting section 18 by a certain operation.

Also, the user-designated-information storing section 23 is configured to store, in the case of user cancelling designation of a tag, initialization data indicating cancellation of the favorite, as user-designated information directed to the tag concerned designated via the favorite-tag setting section 22, upon relating it to the user ID and assigning it to the information on tag acquisition history stored in the second archive section 13-2.

Other configurations are substantially the same as the cell observation information processing system 10 of FIG. 8.

According to the cell observation information processing system 10 of FIG. 12, since the narrowing-down criterion is set to be a certain tag preliminarily determined by the user, it is possible to directly retrieve and present, in the form of a list of tags, only favorite tags for each user, which reflect user's will for use, by narrowing down tags using the narrowing-down criterion preliminarily determined by the user oneself.

Other functions and effects are substantially the same as in the case of the cell observation information processing system 10 of FIG. 8.

In the cell observation information processing system 10 of FIG. 12, the user-designated information storing section 23 is configured to store the user-designated information into the second archive section 13-2, which is a file different from the first archive section 13-1 as a file to store the search-tagged observation information. However, the cell observation information processing system 10 of the third embodiment mode may be configured so that the user-designated information storing section 23 stores the user-designated information into an archive section in the same file as the file to store the search-tagged observation information.

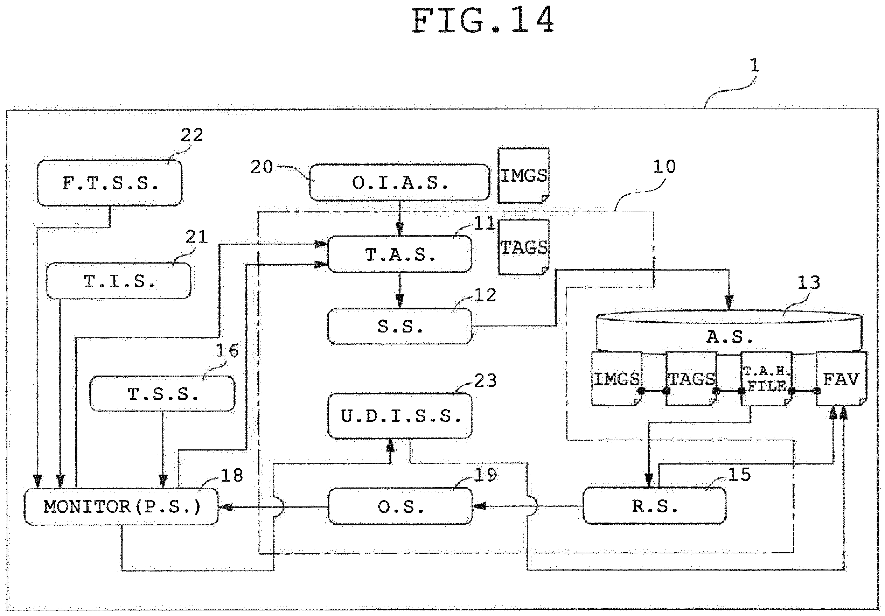

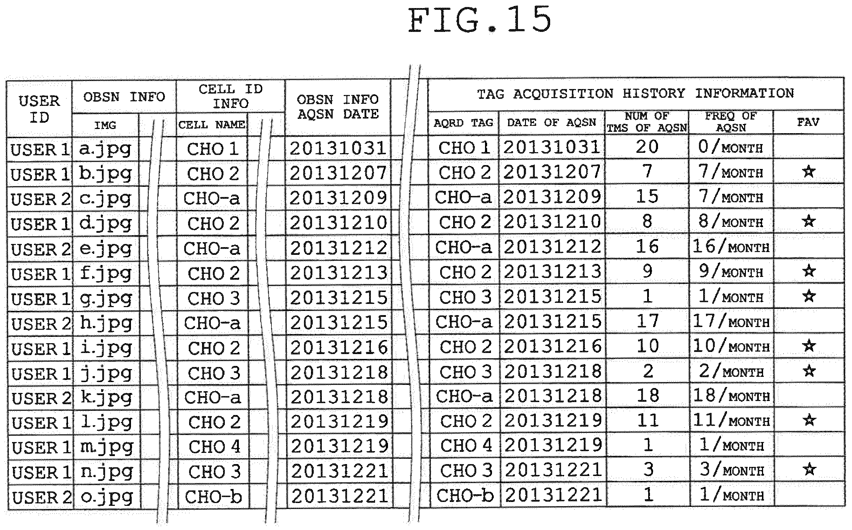

FIG. 14 is an explanatory diagram that shows the configuration of the cell observation information processing system according to one modified example of FIG. 12. FIG. 15 is an explanatory diagram that shows one example of data structure with user-designated favorite information stored in the archive section in the cell observation information processing system of FIG. 14.

In the cell observation information processing system 10 of this modified example, the user-designated-information storing section 23 is configured to store, into the archive section 13, user-designated information upon relating it to the user ID and assigning it to search-tagged observation information to which information on tag acquisition history is assigned. The archive section 13 provides the information on tag acquisition history and the user-designated information, which relate to the search-tagged observation information, as data items building a record, as shown in FIG. 14.

Other configurations and functions and effects are substantially the same as in the case of the cell observation information processing system 10 of FIG. 12.

In the cell observation information processing systems 10 of FIG. 12 and FIG. 14, the user-designated-information storing section 23 is configured to store the user-designated information, upon assigning it to the information on tag acquisition history, into the archive section. However, the cell observation information processing system 10 of the third embodiment mode may be configured so that the user-designated-information storing section 23 stores the user-designated information into the archive section upon assigning it to search-tagged observation information to which information on tag acquisition history is not added.

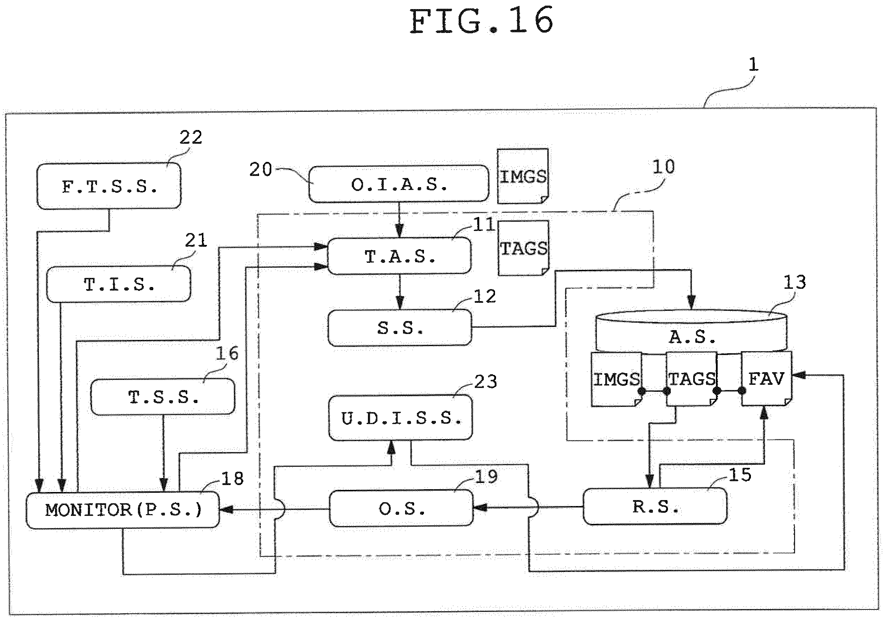

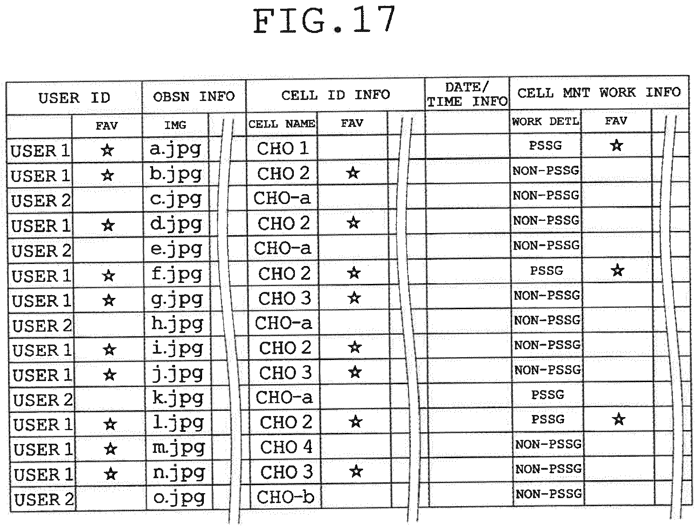

FIG. 16 is an explanatory diagram that shows the configuration of the cell observation information processing system according to another modified example of FIG. 12. FIG. 17 is an explanatory diagram that shows one example of data structure with user-designated information stored in the archive section in the cell observation information processing system of FIG. 16.

In the cell observation information processing system 10 of this modified example, the user-designated-information storing section 23 is configured to store, into the archive section 13, user-designated information upon assigning it to search-tagged observation information.

The favorite-tag setting section 22 is configured to make it possible to designate a certain plurality (of kinds) of tags that the user desires to make the retrieving section 15 designate as a plurality of narrowing-down criteria, via an operation by selection, from a list of tags (not shown in the drawings) displayed in the pull-down menu format on the presenting section 18 by a certain operation.

For example, in the case where the user ID "USER 1", the cell names "CHO 2" and "CHO 3", and the information on passage/non-passage "PASSAGE" categorized in user's work for cell maintenance are designated via the favorite-tag setting section 22 as tags that the user having the user ID "USER 1" desires to make the retrieving section 15 designate as a plurality of narrowing-down criteria, the user-designated-information storing section 23 relates a mark formed of a letter, symbol or figure (for example, pentagram) indicating user's favorite, as user-designated information directed to the user ID "USER 1", the cell names "CHO 2" and "CHO 3", and the information on passage/non-passage "PASSAGE" categorized in user's work for cell maintenance, to the user ID "USER 1". Then, as shown in FIG. 17, the mark is put in the column of favorite-information setting concerned in the following records, out of records of search-tagged observation information stored in the archive section 13, to thereby update these records.
* the column of favorite-information setting directed to user ID in every record carrying the user ID "USER 1".
* the column of favorite-information setting directed to cell name in every record carrying the user ID "USER 1" and the cell name "CHO 2" or "CHO 3"
* the column of favorite-information setting directed to information on passage/non-passage categorized in work in every record carrying the user ID "USER 1" and the information on passage/non-passage "PASSAGE" categorized in user's work for cell maintenance

Also, in the cell observation information processing system 10 of FIG. 16, the retrieving section 15 narrows down the records of search-tagged observation information stored in the archive section 13 to those given the mark (pentagram) indicating the favorite by the user-designated information storing section 23 in the column of favorite-information setting concerned, and retrieves the user ID "USER 1", the cell names "CHO 2" and "CHO 3", and the information on passage/non-passage "PASSAGE" categorized in user' s work for cell maintenance in the form of respective lists of tags.

The favorite-tag setting section 22 is configured also to allow cancellation of designation with respect to at least one kind of tags for which the user desires cancellation of designation, via an operation by selection, from a list (not shown in the drawings) of tags already designated as "a plurality (of kinds) of tags that the user desires to make the retrieving section 15 designate as narrowing-down criteria" in a tag designation cancelling screen displayed for example in the pull-down menu format on the presenting section 18 by a certain operation.

Also, the user-designated-information storing section 23 is configured to store, in the case of user cancelling designation of a tag, initialization data indicating cancellation of the favorite, as user-designated information directed to the tag concerned designated via the favorite-tag setting section 22, upon relating it to the user ID and assigning it to the records of search-tagged observation information stored in the archive section 13.

Other configurations are substantially the same as the cell observation information processing systems 10 of FIG. 12 and FIG. 14.

According to the cell observation informationprocessing system 10 of FIG. 16, since the configuration is made so that the retrieving section 15 can designate a plurality of narrowing-down criteria and so that, out of a plurality of narrowing-down criteria, a designated narrowing-down criterion can be preliminarily determined by the user, it is possible to select a narrowing-down criterion that can be marked by the user as a favorite in accordance with the user's need, and to facilitate listing of only tags that are necessary for the user.

Other functions and effects are substantially the same as in the cases of the cell observation information processing systems 10 of FIG. 12 and FIG. 14.

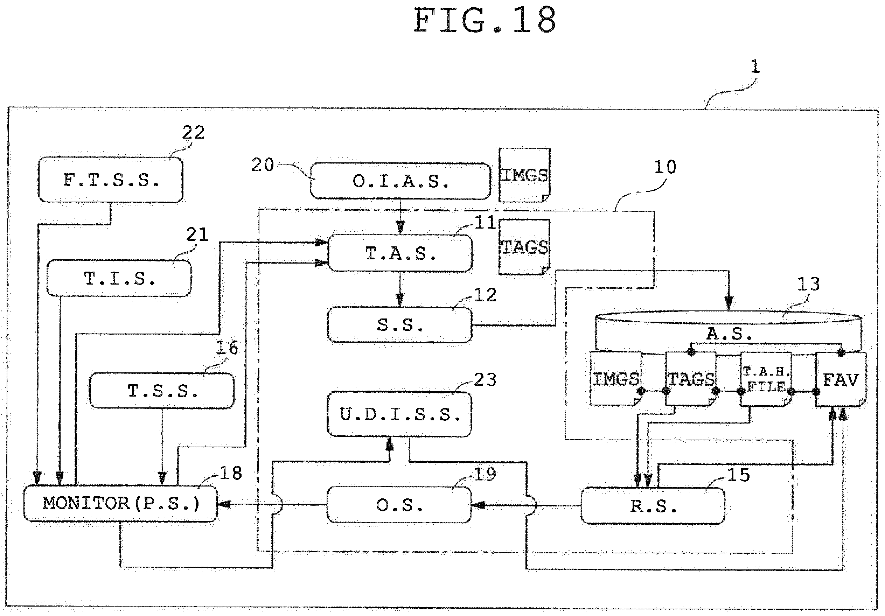

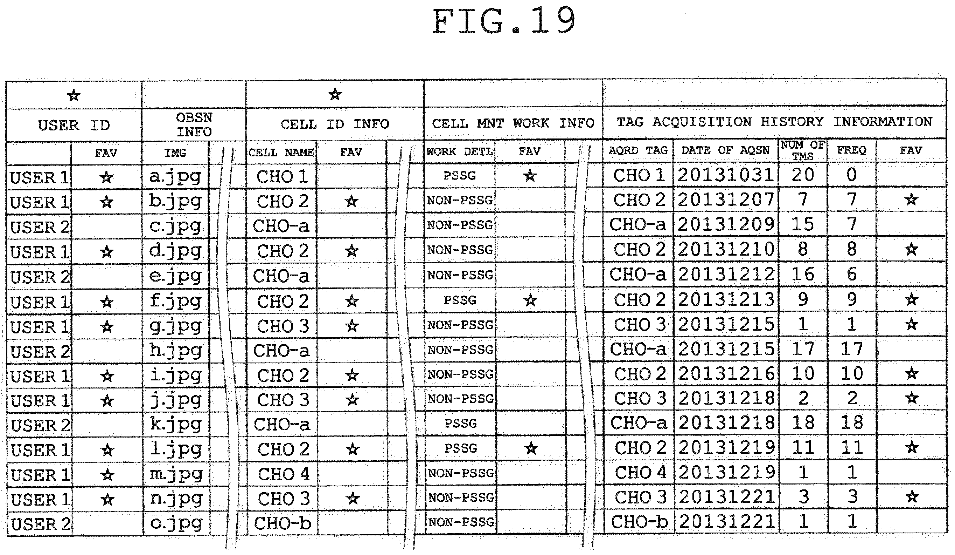

FIG. 18 is an explanatory diagram that shows the configuration of the cell observation information processing system according to still another modified example of FIG. 12. FIG. 19 is an explanatory diagram that shows one example of data structure with user-designated information stored in the archive section in the cell observation information processing system of FIG. 18.

In the cell observation information processing system 10 of this modified example, the configuration is made so that the retrieving section 15 can designate, as a plurality of narrowing-down criteria, in addition to tags, categories tags belong to such as user identifying information, information on user's work for cell maintenance, and information on history of tag acquisition by the tag acquiring section regarding tags that are possible objects of retrieval by the retrieving section. Further, out of the plurality of narrowing-down criteria, a designated narrowing-down criterion has been preliminarily determined by the user.

To be specific, similar to the example of FIG. 16, for example, the favorite-tag setting section 22 makes it possible to designate a certain plurality (of kinds) of tags that the user desires to make the retrieving section 15 designate as a plurality of narrowing-down criteria, via an operation by selection, from a list of tags (not shown in the drawings) displayed in the pull-down menu format on the presenting section 18 by a certain operation. In addition, the favorite-tag setting section 22 is configured to make it possible to designate a category that the user desires to make the retrieving section 15 designate as a narrowing-down criterion, via an operation by selection, among categories tags belong to such as user identifying information, information on user's work for cell maintenance, and information on history of tag acquisition by the tag acquiring section regarding tags that are possible objects of retrieval by the retrieving section, which are displayed in the pull-down menu format on the presenting section 18 by a certain operation as narrowing-down criteria (not shown in the drawings).

In the cell observation information processing system 10 of FIG. 18, the user-designated-information storing section 23 is configured to store user-designated information directed to a certain tag designated via a favorite-tag setting section 22, upon relating it to the user ID and assigning it to records of search-tagged observation information to which information on tag acquisition history is assigned, which are stored in the archive section 13, as well as to store user-designated information directed to a certain category, into which tags fall, designated via a favorite-tag setting section 22, upon relating it to the user ID and assigning it to records of search-tagged observation information to which information on tag acquisition history is assigned, which are stored in the archive section 13.

For example, let us suppose that the user ID "USER 1", the cell names "CHO 2" and "CHO 3", the information on passage/non-passage "PASSAGE" categorized in user's work for cell maintenance, the acquired tags "CHO 2" and "CHO 3" categorized in information on tag acquisition history have been preliminarily selected by the user having the user ID "USER 1" via the favorite-tag setting section 22 as tags that the user desires to make the retrieving section 15 designate as narrowing-down criteria. Let us further suppose that the user-designated-information storing section 23 has related a mark formed of a letter, symbol or figure (for example, pentagram) indicating user's favorite, as user-designated information directed to each of the user ID "USER 1", the cell names "CHO 2" and "CHO 3", the information on passage/non-passage "PASSAGE" categorized in user's work for cell maintenance, and the acquired tags "CHO 2" and "CHO 3" categorized in information on tag acquisition history, to the user ID "USER 1", and has put the mark in the column of favorite-information setting concerned in records of search-tagged observation information to which information on tag acquisition history is assigned and which are stored in the archive section 13, to thereby update these records.

Here, let us still further suppose, for example that "USER ID" and "CELL IDENTIFYING INFORMATION" have been preliminarily designated by the user having the user ID "USER 1" via the favorite-tag setting section 22 as categories tags belong to that the user desires to make the retrieving section 15 designate as narrowing-down criteria. Further, let us suppose that the user-designated-information storing section 23 has related the mark indicating the favorite, as user-designated information directed to each of "USER ID" and "CELL IDENTIFYING INFORMATION", and has put the mark in the column of favorite-information setting concerned in records of search-tagged observation information to which information on tag acquisition history is assigned and which are stored in the archive section 13, to thereby update these records. FIG. 19 shows one example of data structure with user-designated information stored in the archive section 13 at that time.

In this case, the retrieving section 15 narrows down records stored in the archive section 13 to the records given the mark (pentagram) indicating the favorite under the categories "USER ID" and "CELL LEVEL INFORMATION", to which the mark (pentagram) indicating the favorite is assigned by the user-designated information storing section 23, and retrieves the user ID "USER 1" and the cell names "CHO 2" and "CHO 3" in the form of respective lists of tags.

Also, let us further suppose, for example that "USER ID" and "INFORMATION ON PASSAGE/NON-PASSAGE CATEGORIZED IN USER'S WORK FOR CELL MAINTENANCE" instead of "CELL IDENTIFYING INFORMATION" have been preliminarily designated by the user having the user ID "USER 1" via the favorite-tag setting section 22 as categories tags belong to that the user desires to make the retrieving section 15 designate as narrowing-down criteria. Further, let us suppose that the user-designated-information storing section 23 has related the mark indicating the favorite, as user-designated information directed to each of "USER ID" and "INFORMATION ON PASSAGE/NON-PASSAGE CATEGORIZED IN USER'S WORK FOR CELL MAINTENANCE", and has put the mark in the column of favorite-information setting concerned in records of search-tagged observation information to which information on tag acquisition history is assigned and which are stored in the archive section 13, to thereby update these records.

In this case, the retrieving section 15 narrows down records stored in the archive section 13 to the records given the mark (pentagram) indicating the favorite under the categories "USER ID" and "INFORMATION ON PASSAGE/NON-PASSAGE CATEGORIZED IN USER'S WORK FOR CELL MAINTENANCE", to which the mark (pentagram) indicating the favorite is assigned by the user-designated information storing section 23, and retrieves the user ID "USER 1" and the information on passage/non-passage categorized in user's work for cell maintenance "PASSAGE" in the form of respective lists of tags.

The favorite-tag setting section 22 is configured also to allow cancellation of designation with respect to at least one kind of tags or categories tags belong to for which the user desires cancellation of designation, via an operation by selection, from a list (not shown in the drawings) of tags or categories tags belong to that have been already designated as "a plurality (of kinds) of tags or categories tags belong to that the user desires to make the retrieving section 15 designate as narrowing-down criteria" in a tag designation cancelling screen displayed for example in the pull-down menu format on the presenting section 18 by a certain operation.

Also, the user-designated-information storing section 23 is configured to store, in the case of user cancelling designation of a tag or a category tags belong to, initialization data indicating cancellation of the favorite, as user-designated information directed to the tag or the category tags belong to concerned designated via the favorite-tag setting section 22, upon relating it to the user ID and assigning it to records of search-tagged observation information to which information on tag acquisition history is assigned, stored in the archive section 13.

Other configurations are substantially the same as the cell observation information processing systems 10 of FIG. 12, FIG. 14 and FIG. 16.

According to the cell observation informationprocessing system 10 of FIG. 18, since the configuration is made so that the retrieving section can designate a plurality of narrowing-down criteria and so that, out of a plurality of narrowing-down criteria, a designated narrowing-down criterion can be preliminarily determined by the user, it is possible to select a narrowing-down criterion that can be marked by the user as a favorite in accordance with the user's need, and to facilitate listing of only tags that are necessary for the user.

Further, since the configuration is made so that a user can preliminarily determine, as a plurality of narrowing-down criteria, categories tags belong to such as user identifying information, information on user's work for cell maintenance, and information on history of tag acquisition by the tag acquiring section regarding tags that are possible objects of retrieval by the retrieving section, the choice of narrowing-down criteria that can be marked by the user as favorites is widened in accordance with the user's need, and to much facilitate listing of only tags necessary for the user.

Other functions and effects are substantially the same as in the cases of the cell observation information processing systems 10 of FIG. 12, FIG. 14 and FIG. 16.

In this way, according to the several embodiment modes of the present invention, it is possible to attain a cell observation information processing system, a cell observation information processing method, a cell observation information processing program, an archive section provided for the cell observation information processing system, and an apparatus provided for the cell observation information processing system, each of which can save, as much as possible, operation effort and time of the user to acquire tags to be assigned to observation information on cells.

While the embodiment modes of the cell observation information processing system according to the present invention are explained above, the cell observation information processing system may be configured of a cell observation information processing program that makes a computer provided in the installation 1 function as: a tag acquiring means that acquires, as search tags for searching for observation information that has been acquired via an observation information acquiring section in response to an acquisition order at one time point to contain items carrying cell observation results such as a cell image, activity data indicating cell activity by a number of cells, a cell confluency, a morphology, a viability or so. , and an observation title, at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; a storingmeans that assigns, as search tags, the tags acquired by the tag acquiring section to the observation information, which has been acquired in response to an acquisition order at each time point, and that stores search-tagged observation information, to which the search tags have been assigned, in an archive section; a retrieving means that retrieves from the archive section a list of tags as possible search tags; and an output means that outputs the list of tags retrieved by the retrieving section to a presenting section; wherein the retrieving means designates a narrowing-down criterion for narrowing down tags to be retrieved and retrieves the list of tags as narrowed down by the designated narrowing-down criterion, and the tag acquiring means acquires a tag selected by the user from the list of tags presented by the presenting section.

Alternatively, the cell observation information processing system according to the embodiment modes of the present invention may have, other than the configuration in which a cell observation information processing program is provided in a computer provided in the installation 1, for example a configuration as recorded in a recordingmedium readable by a computer provided in the installation 1.

The cell observation information processing system according to the embodiment modes of the present invention is not limited to the configuration in which the tag acquiring section 11, the storing section 12, the retrieving section 15 and the output section 19 are provided in one installation 1 together with the archive section 13, the presenting section 18, the tag selecting section 16, the tag input section 21 and the observation information acquiring section 20 as shown in FIG. 2, but may be configured so that, for example as shown in FIGs. 10 and 11 as modified examples, the tag acquiring section 11, the storing section 12, the retrieving section 15 and the output section 19 are separately arranged in a plurality of apparatuses. Also, the cell observation information processing system according to another embodiment mode of the present invention may be configured to include the archive section 13 also together with the tag acquiring section 11, the storing section 12, the retrieving section 15 and the output section 19.

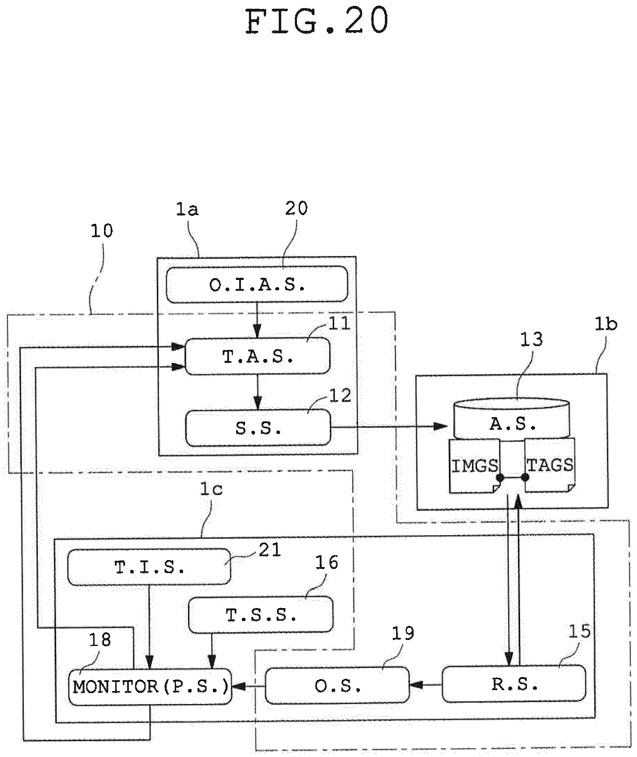

FIG. 20 is an explanatory diagram that shows the configuration of the cell observation information processing system according to one modified example of FIG. 2.

The cell observation information processing system 10 of this modified example is configured so that the tag acquiring section 11 and the storing section 12 are provided in an observation processing apparatus 1a, the archive section 13 is provided in an archive processing apparatus 1b, and the retrieving section 15 and the output section 19 are provided in a monitor apparatus 1c together with the presenting section 18, the tag selecting section 16 and the tag input section 21. The observation information acquiring section 20 is provided in the observation processing apparatus 1a.

The observation processing apparatus 1a is configured, for example of a microscope apparatus provided with an image capture apparatus and a computer.

The archive processing apparatus 1b is configured, for example of a storage unit that stores therein database files.

The monitor apparatus 1c is configured, for example of a display apparatus with a built-in computer provided with a database software.

The observation processing apparatus 1a and the archive processing apparatus 1b, the archive processing apparatus 1b and the monitor apparatus 1c, and the monitor apparatus 1c and the observation processing apparatus 1a are interconnected in network, respectively.

The configurations of the tag acquiring section 11, the storing section 12, the archive section 13, the retrieving section 15, the output section 19, the presenting section 18, the tag selecting section 18, the tag input section 21 and the observation information acquiring section 20 are substantially the same as the configurations in the first embodiment mode shown in FIG. 2.

In the cell observation information processing system 10 of FIG. 20 thus configured, at the stage of storing observation information on cells under culture into the archive section 13, the observation information acquiring section 20 provided in the observation processing apparatus 1a acquires observation information in response to an acquisition order at one time point by a user. Then, the tag acquiring section 11 acquires, via network connection, tags selected by the user via the tag selecting section 16, as search tags for searching for the observation information acquired via the observation information acquiring section 20. Then, the storing section 12 assigns the tags acquired by the tag acquiring section 11 to the observation information, and stores the search-tagged observation information into the archive section 13 in the archive apparatus 1b via network connection.

At a time point before the tag acquiring section 11 acquires the tags selected by the user via the tag selecting section 16, the retrieving section 15 provided in the monitor apparatus 1c searches through the archive apparatus 1b via network connection using a designated narrowing-down criterion and retrieves a list of certain tags as narrowed down with the narrowing-down criterion, and the output section 19 outputs the list of tags as narrowed down in the pull-down menu format on the presenting section 18. Then, the user is allowed to select a desired tag from the list of tags via the tag selecting section 16.

According to the cell observation information processing system 10 of FIG. 20, since the tag acquiring section 11, the storing section 12, the retrieving section 15 and the output section 19, which constitute the cell observation information processing system 10, are dividedly provided in the plurality of apparatuses 1a, 1b and 1c, the individual apparatuses can be made small-sized and simple.

It is noted that the tag acquiring section 11 and the storing section 12 may be provided in an apparatus different from the observation information acquiring section 20.

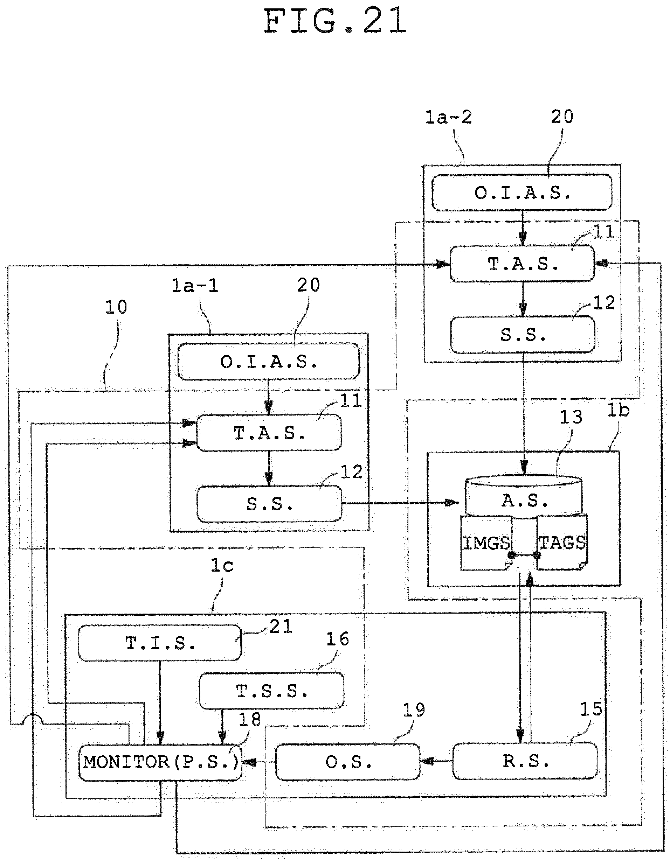

FIG. 21 is an explanatory diagram that shows the configuration of the cell observation information processing system according to another modified example of FIG. 2.

The cell observation information processing system 10 of this modified example is configured so that a plurality of observation processing apparatuses 1a-1 to 1a-n (in FIG. 21, n = 2 for convenience' sake) acquire observation information, and the observation information acquired by the respective observation processing apparatuses 1a-1 to 1a-n are configured to be managed in bulk.

To be specific, in the cell image observation processing system 10 of FIG. 21, each of the plurality of observation processing apparatuses 1a-1 to 1a-n is provided with an observation information acquiring section 20 and a storing section 12.

Other configurations are substantially the same as in the case of the cell information processing system 10 of FIG. 20.

In the cell observation information processing system 10 of FIG. 21 thus configured, at the stage of storing observation information on cells under culture into the archive section, in each of the observation processing apparatuses 1a-1 to 1a-2, the observation information acquiring section 20 acquires observation information in response to an acquisition order by a user at one time point, the tag acquiring section 11 acquires search tags for searching observation information acquired via the observation information acquiring section 20, and the storing section 12 assigns the search tags acquired by the tag acquiring section 11 to the observation information and stores the search-tagged observation information into the archive section 13 in the archive apparatus 1b via each network connection.

The processing procedure at the stage of selecting a tag before the tag acquiring section 11 acquires tags is substantially the same as that in the cell information processing system 10 of FIG. 20.

According to the cell observation information processing system of FIG. 21, since the configuration is made so that the plurality of observation processing apparatuses 1a-1 to 1a-n (in FIG. 21, n = 2 for convenience' sake) acquire observation information and the observation information acquired by the respective observation processing apparatuses 1a-1 to 1a-n are managed in bulk, without being tied to the location of a particular observation processing apparatus 1a-n, a user can acquire observation information via an observation processing apparatus provided at a desired location and centrally record the search-tagged observation information acquired via the individual observation processing apparatuses into the single archive section 13, to enjoy much improved convenience.

Further, since the configuration is made so that the observation data acquired by the plurality of observation processing apparatuses 1a-1 to 1a-n are managed in bulk, if each of the observation processing apparatuses is configured to be small-sized and light-weighted as a mobile unit for exclusive use by a particular user, each user can acquire observation information on cells at any place, to enjoy much improved convenience.

Other configurations and functions and effects are substantially the same as in the case of the modified example of FIG. 20.

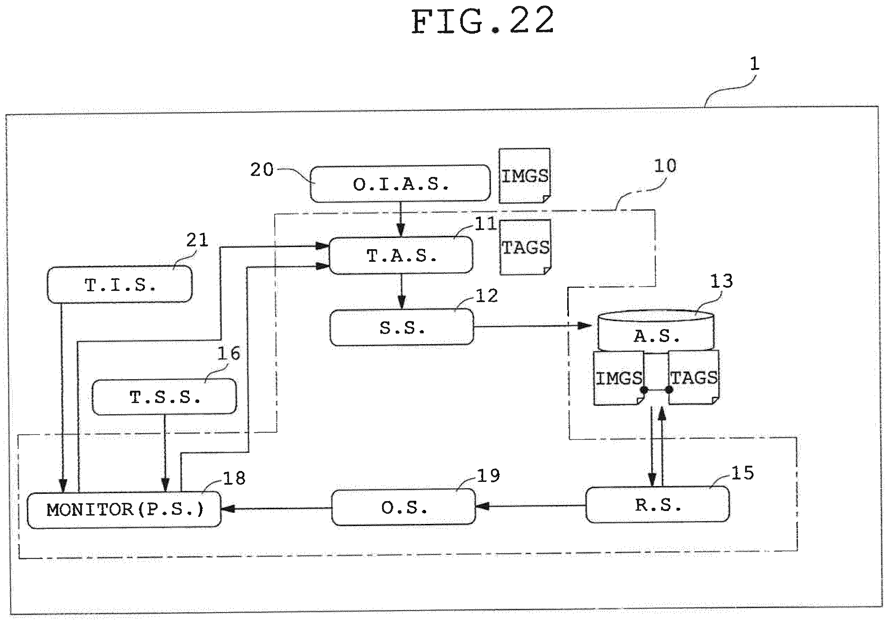

FIG. 22 is an explanatory diagram that shows the configuration of the cell observation information processing system according to still another modified example of FIG. 2.

The cell observation information processing system 10 of this modified example is configured of the cell observation information processing system of FIG 2 further provided with the presenting section 18.

The presenting section 18 displays the list of tags retrieved by the retrieving section 15 and outputted by the output section 19, for example in the pull-down menu format shown in FIG. 3, on a display surface such as a monitor.

Other configurations and functions and effects are substantially the same as the configurations and functions and effects of the first embodiment mode shown in FIG. 2.

While the above explanations are made on the embodiment modes and their modified examples of the present invention, the present invention is not limited to the exact configurations as described in the embodiment modes and their modified examples. At the stage of reduction into practice, components can be embodied as being modified within the scope of not changing the essential concept of the invention. In addition, by appropriately combining a plurality of components described in the embodiment modes and their modified examples together, various inventions can be introduced. For example, some components may be removed from all of the components described in the embodiment modes and their modified examples or components described indifferent embodiment modes and their modified examples can be appropriately combined together. In this way, various modifications and applications are possible within the scope of not changing the essential concept of the invention.

### INDUSTRIAL APPLICABILITY

The cell observation information processing system, the cell observation information processing method, the cell observation information processing program, the archive section provided for the cell observation information processing system, and the apparatuses provided for the cell observation informationprocessing system are useful in fields where there is a demand for condition management of living object that changes as time passes, besides the fields where there is a demand for condition management of cells used for research of living obj ect upon use of observation information on the cells acquired by an observation information acquiring apparatus such as a microscope.

### DESCRIPTION OF THE REFERENCE SYMBOLS

- 1: cell observation information processing installation
- 1a, 1a-1, 1a-2: observation processing apparatus
- 1b: archive processing apparatus
- 1c: monitor apparatus
- 10: cell observation information processing system
- 11: tag acquiring section
- 12: storing section
- 13, 13-1, 13-2: archive section
- 15: retrieving section
- 16: tag selecting section
- 18: presenting section (monitor)
- 19: output section
- 20: observation information acquiring section
- 21: tag input section
- 22: favorite-tag setting section
- 23: user-designated-information storing section

## Claims

1. A cell observation information processing system comprising:
a tag acquiring section that acquires, as search tags for searching for observation information that has been acquired via an observation information acquiring section in response to an acquisition order at one time point to carry a cell observation result including at least one of a cell image and activity data indicating cell activity by at least one of a number of cells, a cell confluency, a morphology, and a viability, at least one of a tag indicating user identifying information for identifying a user who conducts cell observation, a tag indicating cell identifying information for identifying cells under observation, a tag indicating date and time information for identifying a date and time when the observation information was acquired, a tag indicating information on user's work for cell maintenance, a tag indicating apparatus identifying information for identifying an apparatus used in acquiring the observation information, a tag indicating variation information of the activity data, and an image tag for indicating the cell image;
a storing section that assigns, as the search tags, tags acquired by the tag acquiring section to each of the observation information, which has been acquired in response to an acquisition order at each time point, and that stores search-tagged observation information, to which the search tags have been assigned, into an archive section;
a retrieving section that retrieves from the archive section a list of tags as possible search tags; and
an output section that outputs the list of tags retrieved by the retrieving section to a presenting section,
**characterized in that**
the retrieving section designates a narrowing-down criterion for narrowing down tags to be retrieved and retrieves the list of tags as a result of narrowing down with the designated narrowing-down criterion, and
the tag acquiring section acquires a tag selected by the user from the list of tags presented by the presenting section.

2. The cell observation information processing system according to claim 1,
**characterized in that**
the narrowing-down criterion is the user identifying information.

3. The cell observation information processing system according to claim 2,
**characterized in that**
the retrieving section narrows down tags indicating the cell identifying information using the user identifying information.

4. The cell observation information processing system according to claim 3,
**characterized in that**
the cell identifying information includes at least one of a cell name, a cell kind, cell level information indicating whether the cells are of a parent cell line to be cultured or of a subculture separated from and cut out of the parent cell line, and a passage number of the cells.

5. The cell observation information processing system according to claim 3,
**characterized in that**
the narrowing-down criterion is the user identifying information and information on history of tag acquisition by the tag acquiring section with respect to a tag indicating the cell identifying information.

6. The cell observation information processing system according to claim 5,
**characterized in that**
the information on history of tag acquisition by the tag acquiring section with respect to the tag indicating the cell identifying information includes a latest date of acquisition and a total number of times of acquisition up to a present time or a number of times of acquisition during a predetermined retrospective period from the present time.

7. The cell observation information processing system according to claim 6,
**characterized in that**
by use of the information on history of tag acquisition, the retrieving section narrows down tags indicating the cell identifying information, which are possible objects of retrieval, to tags having a latest date of acquisition later than a predetermined date or to tags having a value greater than a predetermined value with respect to a total number of times of acquisition up to the present time or a number of times of acquisition during a predetermined retrospective period of time from the present time.

8. The cell observation information processing system according to claim 1,
**characterized in that**
the narrowing-down criterion is information on history of tag acquisition by the tag acquiring section.

9. The cell observation information processing system according to claim 8,
**characterized in that**
the information on history of tag acquisition by the tag acquiring section includes a latest date of acquisition and a total number of times of acquisition up to a present time or a number of times of acquisition during a predetermined retrospective period from the present time.

10. The cell observation information processing system according to claim 9,
**characterized in that**
by use of the information on history of tag acquisition, the retrieving section narrows down tags, which are possible objects of retrieval, to tags having a latest date of acquisition later than a predetermined date or to tags having a value greater than a predetermined value with respect to a total number of times of acquisition up to the present time or a number of times of acquisition during a predetermined retrospective period of time from the present time.

11. The cell observation information processing system according to claim 8,
**characterized in that**
the storing section further stores the information on history of tag acquisition into the archive section.

12. The cell observation information processing system according to claim 11,
**characterized in that**
the storing section stores, into the archive section, the information on history of tag acquisition upon relating it to at least one of the cell identifying information and the user-identifying information.

13. The cell observation information processing system according to claim 12,
**characterized in that**
the storing section stores the information on history of tag acquisition, which is related to at least one of the cell identifying information and the user-identifying information, into a file different from a file for storing the search-tagged observation information therein, in the archive section.

14. The cell observation information processing system according to claim 12,
**characterized in that**
the storing section assigns the information on history of tag acquisition, which is related to at least one of the cell identifying information and the user-identifying information, as a tag for designating a narrowing-down criterion, to the search-tagged observation information, and stores the search-tagged observation information to which the tag for designating a narrowing-down criterion is assigned into a file for storing the search-tagged observation information therein, in the archive section.

15. The cell observation information processing system according to claim 12,
**characterized in that**
the retrieving section designates the information on history of tag acquisition, which is related to at least one of the cell identifying information and the user identifying information by the storing section, as the narrowing-down criterion, and narrows down tags of at least one of the cell identifying information and the user identifying information using the narrowing-down criterion as designated.

16. The cell observation information processing system according to claim 1,
**characterized in that**
the retrieving section is configured to be able to designate a plurality of narrowing-down criteria and, out of the plurality of narrowing-down criteria, a designated narrowing-down criterion has been preliminarily determined by a user.

17. The cell observation information processing system according to claim 16,
**characterized in that**
the plurality of narrowing-down criteria are the user identifying information, the information on user's work for cell maintenance, and the information on history of tag acquisition by the tag acquiring section with respect to tags which are possible objects of retrieval by the retrieving section.

18. The cell observation information processing system according to claim 1,
**characterized in that**
the narrowing-down criterion is a predetermined tag preliminarily determined by the user.

19. The cell observation information processing system according to claim 16,
**characterized in that**
the cell observation information processing system further comprises a user-designated-information storing section that stores, into the archive section, user-designated information directed to certain tags preliminarily determined by the user.

20. The cell observation information processing system according to claim 19,
**characterized in that**
the user-designated-information storing section stores the user-designated information, upon relating the user-designated information to at least one of the cell identifying information and the user identifying information, into the archive section.

21. The cell observation information processing system according to claim 20,
**characterized in that**
the user-designated-information storing section stores the user-designated information related to at least one of the cell identifying information and the user identifying information, into a file different from a file for storing the search-tagged observation information therein, in the archive section.

22. The cell observation information processing system according to claim 20,
**characterized in that**
the user-designated-information storing section assigns the user-designated information related to at least one of the cell identifying information and the user identifying information, as a tag for designating a narrowing-down criterion, to the search-tagged observation information, and stores the search-tagged observation information to which the tag for designating a narrowing-down criterion is assigned into a file for storing the search-tagged observation information therein, in the archive section.

23. The cell observation information processing system according to claim 20,
**characterized in that**
the retrieving section designates the user-designated information, which is related to at least one of the cell identifying information and the user identifying information by the user-designated information storing section, as the narrowing-down criterion, and narrows down tags of at least one of the cell identifying information and the user identifying information using the narrowing-down criterion as designated.

24. The cell observation information processing system according to claim 1,
**characterized in that**
the narrowing-down criterion is information on cell kind categorized in the cell identifying information, and
the retrieving section narrows down tags indicating the cell name categorized in the cell identifying information, by use of the information on cell kind.

25. The cell observation information processing system according to claim 1,
**characterized in that**
the narrowing down criterion is information on passage or non-passage categorized in the user's work for cell maintenance.

26. The cell observation information processing system according to claim 25,
**characterized in that**
the retrieving section narrows down tags indicating cell level information, which is for identifying whether the cells are of a parent cell line to be cultured or of a subculture separated from and cut out of the parent cell line, categorized in the cell identifying information, by use of information on passage or non-passage categorized in the user's work for cell maintenance.

27. A cell observation information processing method comprising:
a tag acquiring step for acquiring, as search tags for searching for observation information that has been acquired via an observation information acquiring section in response to an acquisition order at one time point to carry a cell observation result including at least one of a cell image and activity data indicating cell activity by at least one of a number of cells, a cell confluency, a morphology, and a viability, at least one of a tag indicating user identifying information for identifying a user who conducts cell observation, a tag indicating cell identifying information for identifying cells under observation, a tag indicating date and time information for identifying a date and time when the observation information was acquired, a tag indicating information on user's work for cell maintenance, a tag indicating apparatus identifying information for identifying an apparatus used in acquiring the observation information, a tag indicating variation information of the activity data, and an image tag for indicating the cell image;
a storing step for assigning, as the search tags, tags acquired at the tag acquiring step to each of the observation information, which has been acquired in response to an acquisition order at each time point, and storing search-tagged observation information, to which the search tags have been assigned, into an archive section;
a retrieving step for retrieving from the archive section a list of tags as possible search tags; and
an outputting step for outputting the list of tags retrieved by the retrieving section to a presenting section,
**characterized in that**
the retrieving step includes designating a narrowing-down criterion for narrowing down tags to be retrieved and retrieving the list of tags as a result of narrowing down with the designated narrowing-down criterion, and
the tag acquiring step includes acquiring a tag selected by the user from the list of tags presented by the presenting section.

28. A cell observation information processing program, **characterized by** making a computer function as:
a tag acquiring means that acquires , as search tags for searching for observation information that has been acquired via an observation information acquiring section in response to an acquisition order at one time point to carry a cell observation result including at least one of a cell image and activity data indicating cell activity by at least one of a number of cells, a cell confluency, a morphology, and a viability, at least one of a tag indicating user identifying information for identifying a user who conducts cell observation, a tag indicating cell identifying information for identifying cells under observation, a tag indicating date and time information for identifying a date and time when the observation information was acquired, a tag indicating information on user's work for cell maintenance, a tag indicating apparatus identifying information for identifying an apparatus used in acquiring the observation information, a tag indicating variation information of the activity data, and an image tag for indicating the cell image;
a storing means that assigns, as the search tags, tags acquired by the tag acquiring means to each of the observation information, which has been acquired in response to an acquisition order at each time point, and that stores search-tagged observation information, to which the search tags have been assigned, into an archive section;
a retrieving means that retrieves from the archive section a list of tags as possible search tags; and
an output means that outputs the list of tags retrieved by the retrieving means to a presenting section,
**characterized in that**
the retrieving means designates a narrowing-down criterion for narrowing down tags to be retrieved and retrieves the list of tags as a result of narrowing down with the designated narrowing-down criterion, and
the tag acquiring means acquires a tag selected by the user from the list of tags presented by the presenting section.

29. The archive section provided for the cell observation information processing system according to claim 1,
**characterized in that**:
the archive section has one or more records of the search-tagged observation information,
each of the records is created in response to image capture of cells at one time point, and
the observation information corresponding to the image capture of cells at one time point and the search tags for searching for the observation information are interrelated.

30. A plurality of apparatuses provided for the cell observation information processing system according to claim 1,
**characterized in that**:
the tag acquiring section, the storing section, the retrieving section and the output section are separately arranged in the plurality of the apparatuses.

31. The cell observation information processing system according to claim 1,
further comprising the presenting section that presents the list of tags retrieved by the retrieving section to the user.
